# EUROPEAN PATENT SPECIFICATION

(11) **EP 3 618 825 B1**
(45) Date of publication and mention of the grant of the patent: **21.10.2026**
(21) Application number: 18794455.8
(22) Date of filing: 02.05.2018
(51) Int. Cl.: A61K 31/255, A61P 35/00, A61K 9/00, A61K 9/19, A61K 47/69, A61K 31/724

(54) **COMPOSITION CONTAINING CYCLODEXTRIN AND BUSULFAN**
ZUSAMMENSETZUNG MIT CYCLODEXTRIN UND BUSULFAN
COMPOSITION CONTENANT DE LA CYCLODEXTRINE ET DU BUSULFAN

(30) Priority: 03.05.2017 US 201762500970 P
(43) Date of publication of application: 11.03.2020
(73) Proprietor: CyDex Pharmaceuticals, Inc., Lawrence, KS 66047 (US)
(72) Inventor: PIPKIN, James, D., Lawrence, KS 66047 (US)
(74) Representative: Heller, Benjamin Henry
(86) International application number: PCT/US2018/030728
(87) International publication number: WO 2018/204535

(56) References cited:
- WO-A1-92/02256
- WO-A1-95/08991
- NZ-A- 568 288
- US-A1- 2005 215 520
- US-A1- 2005 255 164
- NOUR EDDINE GHERMANI ET AL: "Molecular Reactivity of Busulfan Through Its Experimental Electrostatic Properties in the Solid State", PHARMACEUTICAL RESEARCH, 1 April 2004 (2004-04-01), New York, pages 598 - 607, XP055758858, Retrieved from the Internet <URL:10.1023/B:PHAM.0000022406.04888.f1> [retrieved on 20201210], DOI: 10.1023/B:PHAM.0000022406.04888.f1
- MENUEL, S. ET AL.: "Synthesis and inclusion ability of a bis-beta-cyclodextrin pseudo- cryptand towards Busulfan anticancer agent", TETRAHEDRON, vol. 63, no. 7, 2007, pages 1706 - 1714, XP005829486
- PORWANSKI, S. ET AL.: "Bis-β-cyclodextrinyl- and bis-cellobiosyl-diazacrowns: synthesis and molecular complexation behaviors toward Busulfan anticancer agent and two basic aminoacids", TETRAHEDRON, vol. 65, no. 31, 27 March 2009 (2009-03-27), pages 6196 - 6203, XP055545029

## Description

### BACKGROUND

This application claims the benefit of U.S. Provisional Application No. 62/500970, filed May 3, 2017.

### Field

The present invention relates to formulations containing busulfan and sulfoalkyl ether cyclodextrin, a process of preparing and method of using the same.

### Description of the Related Art

Busulfan [1,4-bis-(methanesulfonoxyl)butane], is an alkylating agent used extensively for its antitumor properties, characterized in the early 1950s by Galton et al. for the treatment of chronic myeloid leukemia ( CML). Its poor aqueous solubility, stability and unpleasant side effects (including significant gastric irritation, nausea and vomiting) mean that oral dosage forms show significant variability in bioavailability. In addition, the drug is rapidly metabolized by the liver and can cause severe hepatotoxicity, which can be dose limiting in high dose regimens. Certain solvent used to dissolve busulfan can cause liver damage and put the patient's long-term treatment goals and quality of life at risk.

### Cited Documents

NZ568288 describes compositions comprising a a cyclodextrin and a nephrotoxic anticancer drug. US2005/215520 describes a complex of organic medicines and beta-cyclodextrin derivatives and its preparing process. US2005/255164 describes solid nano pharmaceutical formulation and preparation method thereof. WO95/08991 describes parenteral busulfan for treatment of malignant disease. WO92/02256 describes cyclodextrin complexes for neuraxial administration of drugs. Nour Eddine Ghermani et al., Pharmaceutical research, 2004, 598 - 607, describes molecular reactivity of busulfan through its experimental electrostatic properties in the solid state.

### SUMMARY

The scope of the present invention is defined by the appended claims.

Some embodiments relate to a pharmaceutical composition, comprising a solid that comprises: busulfan; and a cyclodextrin; wherein at least 25% of the busulfan in the composition is complexed with the cyclodextrin, wherein the molar ratio of cyclodextrin to busulfan is less than 12, wherein the cyclodextrin is a compound, or a mixture thereof, of Formula 1: 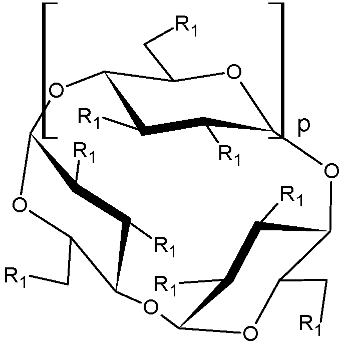 or a pharmaceutically acceptable salt thereof, wherein:
p is 4, 5, or 6, optionally wherein p is 5;
each R₁ is independently -OH or -O-( C₁-C₈ alkylene)-SO₃⁻-T, wherein at least one R₁ is -O-(C₁-C₈ alkylene)-SO₃⁻-T; and
T is hydrogen or a pharmaceutically acceptable cation;
provided that at least one R₁ is -OH.

Some embodiments relate to a pharmaceutical composition, comprising a clear aqueous solution that includes busulfan, wherein the concentration of busulfan is in the range of about 0.3 mg/ml to about 3 mg/ml; and cyclodextrin, wherein the molar ratio of cyclodextrin to busulfan is less than 12.

Some embodiments relate to a pharmaceutical composition, comprising a clear aqueous solution that comprises busulfan, wherein the concentration of busulfan is in the range of about 0.3 mg/ml to 3 mg/ml; and a sulfoalkyl ether cyclodextrin, wherein the molar ratio of cyclodextrin to busulfan is less than about 12.

Some embodiments relate to a reconstituted solution obtained by adding a pharmaceutically acceptable solvent to the composition described herein.

Some embodiments relate to a sterilized container comprising the composition described herein.

Some embodiments relate to a process for preparing a busulfan composition, comprising:
combining busulfan and an organic solvent to provide a clear solution;
combining the clear solution with a cyclodextrin to provide a first mixture wherein combining the clear solution with a cyclodextrin comprises combining the clear solution with an aqueous cyclodextrin solution;
removing the organic solvent from the first mixture to provide a second mixture; and
   drying the first mixture to provide the busulfan composition;
wherein the molar ratio of cyclodextrin to busulfan in the busulfan composition is less than 12,
wherein the cyclodextrin is a compound, or a mixture thereof, of Formula 1: 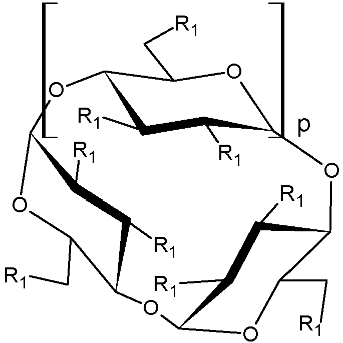
or a pharmaceutically acceptable salt thereof, wherein:
   p is 4, 5, or 6, optionally wherein p is 5;
   each R₁ is independently -OH or -O-( C₁-C₈ alkylene)-SO₃⁻-T, wherein at least one R₁ is -O-(C₁-C₈ alkylene)-SO₃⁻-T, optionally at least one R₁ is - OCH₂CH₂CH₂CH₂SO₃T or -OCH₂CH₂CH₂SO₃T; and
   T is hydrogen or a pharmaceutically acceptable cation, optionally wherein T is independently hydrogen or sodium;
   provided that at least one R₁ is -OH.

Some embodiments relate to a pharmaceutical composition described herein for use in a method of treatment, comprising; reconstituting the pharmaceutical composition described herein; and administering the reconstituted pharmaceutical composition to a subject in need thereof.

Some embodiments relate to a pharmaceutical composition described herein for use in a method of conditioning a subject for hematopoietic stem-cell transplantation, comprising administering the composition described herein to a subject in need thereof.

Some embodiments relate to a pharmaceutical composition described herein for use in a method of conditioning a subject for bone marrow transplantation, comprising administering the composition described herein to a subject in need thereof.

Some embodiments relate to a pharmaceutical composition described herein for use in a method of treating leukemia, lymphoma, and myeloproliferative disorder, comprising administering the composition described herein to a subject in need thereof.

### BRIEF DESCRIPTION OF THE DRAWINGS

Figure 1 illustrates the results of a phase solubility study of busulfan in sulfoalkylether-β-cyclodextrin solutions.
Figure 2 illustrates the results of a stability study using three busulfan formulations.
Figure 3A illustrates the stability of a first busulfan formulation at 2-8 °C, 25 °C, and 40 °C as well as a third busulfan formulation at 2-8 °C (RF) and 25 °C; and Figure 3B shows the stability of a second busulfan formulation at 2-8 °C(RF), 25 °C, and 40 °C as well as a third busulfan formulation at 2-8 °C (RF) and 25 °C.

### DETAILED DESCRIPTION

The term "pharmaceutically acceptable cation" refers to cations that retain the biological effectiveness and properties of a compound and, which are not biologically or otherwise undesirable for use in a pharmaceutical. Examples of cation include but are not limited to sodium, potassium, lithium, ammonium, calcium, magnesium, iron, zinc, copper, manganese, aluminum, and the like; particularly preferred are the ammonium, potassium, sodium, calcium and magnesium cations. Other types of cations can include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such cations are known in the art, as described in WO 87/05297, Johnston et al., published September 11, 1987.

"The term "pharmaceutically acceptable salt" refers to salts that retain the biological effectiveness and properties of the compounds of the preferred embodiments and, which are not biologically or otherwise undesirable. In many cases, the compounds of the preferred embodiments are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in World Patent Publication 87/05297, Johnston et al., published September 11, 1987.

The term "pharmaceutically-acceptable solvent", as used herein, means one or more compatible solid or liquid filler diluents or encapsulating substances, which are suitable for administration to a mammal. The term "compatible" or "acceptable", as used herein, means that the components of the composition are capable of being commingled with the subject compound, and with each other, in a manner such that there is no interaction, which would substantially reduce the pharmaceutical efficacy of the composition under ordinary use situations. Pharmaceutically-acceptable carriers must, of course, be of sufficiently high purity and sufficiently low toxicity to render them suitable for administration preferably to an animal, preferably mammal being treated.

Some examples of substances, which can serve as pharmaceutically-acceptable carriers or components thereof, are cyclodextrins (e.g., SAE-CD, HAE-CD, and other cyclodextrin derivatives), sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

As used herein, "a major portion of the busulfan" refers to at least at least about 25%, 30%, 35%, 40%, 45%, 50%, 55%, 60%, 65%, 70%, 75%, 80%, 85%, 90%, 95%, or 99% of the busulfan in the composition.

As used herein, "alkyl" refers to a straight or branched hydrocarbon chain that is fully saturated (i.e., contains no double or triple bonds). The alkyl group may have 1 to 20 carbon atoms (whenever it appears herein, a numerical range such as "1 to 20" refers to each integer in the given range; *e.g.,* "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 9 carbon atoms. The alkyl group could also be a lower alkyl having 1 to 4 carbon atoms. The alkyl group may be designated as "C₁₋₄ alkyl" or similar designations. By way of example only, "C₁₋₄ alkyl" indicates that there are one to four carbon atoms in the alkyl chain, i.e., the alkyl chain is selected from the group consisting of methyl, ethyl, propyl, isopropyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like.

A "sulfonyl" group refers to an "-SO₂R" group in which R is selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

The term "mammal" is used in its usual biological sense. Thus, it specifically includes, but is not limited to, primates, including simians (chimpanzees, apes, monkeys) and humans, cattle, horses, sheep, goats, swine, rabbits, dogs, cats, rodents, rats, mice guinea pigs, or the like.

An "effective amount" or a "therapeutically effective amount" as used herein refers to an amount of a therapeutic agent that is effective to relieve, to some extent, or to reduce the likelihood of onset of, one or more of the symptoms of a disease or condition, and includes curing a disease or condition. "Curing" means that the symptoms of a disease or condition are eliminated; however, certain long-term or permanent effects may exist even after a cure is obtained (such as extensive tissue damage).

"Treat," "treatment," or "treating," as used herein refers to administering a pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

The compositions and processes described herein can help to reduce or eliminate the amount of a toxic solvent such as N,N-dimethylacetamide (DMA) and/or nonaqueous such as PEG in the busulfan formulation. The process and compositions described herein can lead to higher drug load requiring a relatively lower amount of cyclodextrin or cyclodextrin derivative. The superior properties of the process and composition described herein help to achieve improved drug tolerance, better drug stability, higher drug exposure, longer duration of therapy, and also more options with flexible handling and improved stability.

The compositions and processes described herein provide high-dose parenteral busulfan therapy for the treatment of various diseases, while solving the problems of the use of toxic solvent and busulfan instability associated with existing busulfan compositions. The busulfan composition described herein shows less drug precipitation over time when compared with other commercially available busulfan formulation. The present technology also provides the opportunity for expanded use in pediatric population due to the reduced solvent toxicity.

The technical information set out below may in some respects go beyond the scope of the present invention, which is defined by the appended claims. The additional technical information is provided to place the present invention in a broader technical context and to illustrate possible related technical developments.

### Cyclodextrin

The term "cyclodextrin" as used herein refers to an α-cyclodextrin, β-cyclodextrin, γ-cyclodextrin, or their respective derivatives, including derivatives in which one or more hydroxy on the cyclodextrin core is replaced with an alkylether, hydroxyalkylether, or sufloalkylether substituent. Examples of cyclodextrin derivative can include but are not limited to the cyclodextrins listed in Tables A to D below. Preferably, the cyclodextrin is a β-cyclodextrin, hydroxypropyl-β-cyclodextrin, or sulfoalkyl ether β-cyclodextrin.

Some exemplary sulfoalkyl ether (SAE)-CD derivatives include:

**Table A.**

| SAEx-α-CD | SAEx-β-CD | SAEx-γ-CD |
|---|---|---|
| (Sulfoethyl ether)x-α-CD | (Sulfoethyl ether)x-β-CD | (Sulfoethyl ether)x-γ-CD |
| (Sulfopropyl ether)x-α-CD | (Sulfopropyl ether)x-β-CD | (Sulfopropyl ether)x-γ-CD |
| (Sulfobutyl ether)x-α-CD | (Sulfobutyl ether)x-β-CD | (Sulfobutyl ether)x-γ-CD |
| (Sulfopentyl ether)x-α-CD | (Sulfopentyl ether)x-β-CD | (Sulfopentyl ether)x-γ-CD |
| (Sulfohexyl ether)x-α-CD | (Sulfohexyl ether)x-β-CD | (Sulfohexyl ether)x-γ-CD |

wherein x denotes the average degree of substitution. In some embodiments, the alkylated cyclodextrins are formed as salts.

Various embodiments of a sulfoalkyl ether cyclodextrin include eicosa-O-(methyl)-6G-O-(4-sulfobutyl)-β-cyclodextrin, heptakis-O-(sulfomethyl)-tetradecakis-O-(3-sulfopropyl)-β-cyclodextrin, heptakis-O-[(1,1-dimethylethyl)dimethylsilyl]-tetradecakis-O-(3-sulfopropyl)-β-cyclodextrin, heptakis-O-(sulfomethyl)-tetradecakis-O-(3-sulfopropyl)-β-cyclodextrin, and heptakis-O-[(1,1-dimethylethyl)dimethylsilyl]-tetradecakis-O-(sulfomethyl)-β-cyclodextrin. Other known alkylated cyclodextrins containing a sulfoalkyl moiety include sulfoalkylthio and sulfoalkylthioalkyl ether derivatives such as octakis-(S-sulfopropyl)-octathio-γ-cyclodextrin, octakis-O-[3-[(2-sulfoethyl)thio]propyl]-β-cyclodextrin], and octakis-S-(2-sulfoethyl)-octathio-γ-cyclodextrin.

In some embodiments, an alkylated cyclodextrin composition of the present invention is a sulfoalkyl ether-β-cyclodextrin composition having an ADS of 2 to 9, 4 to 8, 4 to 7.5, 4 to 7, 4 to 6.5, 4.5 to 8, 4.5 to 7.5, 4.5 to 7, 5 to 8, 5 to 7.5, 5 to 7, 5.5 to 8, 5.5 to 7.5, 5.5 to 7, 5.5 to 6.5, 6 to 8, 6 to 7.5, 6 to 7.1, 6.5 to 7.1, 6.2 to 6.9, or 6.5 per alkylated cyclodextrin, and the remaining substituents are -H.

Some exemplary Alkyl ether cyclodextrin (AE-CD) derivatives include:

**Table B.**

| (Alkylether)_{y}-α-CD | (Alkylether)_{y}-β-CD | (Alkylether)_{y}-γ-CD |
|---|---|---|
| ME_{y}-α-CD | ME_{y}-β-CD | ME_{y}-γ-CD |
| EE_{y}-α-CD | EE_{y}-β-CD | EE_{y}-γ-CD |
| PE_{y}-α-CD | PE_{y}-β-CD | PE_{y}-γ-CD |
| BE_{y}-α-CD | BE_{y}-β-CD | BE_{y}-γ-CD |
| PtE_{y}-α-CD | PtE_{y}-β-CD | PtE_{y}-γ-CD |
| HE_{y}-α-CD | HE_{y}-β-CD | HE_{y}-γ-CD |

wherein ME denotes methyl ether, EE denotes ethyl ether, PE denotes propyl ether, BE denotes butyl ether, PtE denotes pentyl ethyl, HE denotes hexyl ether, and y denotes the average degree of substitution.

Some exemplary hydroxyalkyl ether (HAE)-CD derivatives include:

**Table C.**

| (HAE)z-α-CD | (HAE)z-β-CD | (HAE)z-γ-CD |
|---|---|---|
| HMEz-α-CD | HMEz-β-CD | HMEz-γ-CD |
| HEEz-α-CD | HEEz-β-CD | HEEz-γ-CD |
| HPEz-α-CD | HPEz-β-CD | HPEz-γ-CD |
| HBEz-α-CD | HBEz-β-CD | HBEz-γ-CD |
| HPtEz-α-CD | HPtEz-β-CD | HPtEz-γ-CD |
| HHEz-α-CD | HHEz-β-CD | HHEz-γ-CD |

wherein HME denotes hydroxymethyl ether, HEE denotes hydroxyethyl ether, HPE denotes hydroxypropyl ether, HBE denotes hydroxybutyl ether, HPtE denotes hydroxypentyl ether, HHE denotes hydroxyhexyl ether, and z denotes the average degree of substitution.

The alkylated cyclodextrin can include SAE-CD, HAE-CD, SAE-HAE-CD, HANE-CD, HAE-AE-CD, HAE-SAE-CD, AE-CD, SAE-AE-CD, neutral cyclodextrin, anionic cyclodextrin, cationic cyclodextrin, halo-derivatized cyclodextrin, amino-derivatized cyclodextrin, nitrile-derivatized cyclodextrin, aldehyde-derivatized cyclodextrin, carboxylate-derivatized cyclodextrin, sulfate-derivatized cyclodextrin, sulfonate-derivatized cyclodextrin, mercapto-derivatized cyclodextrin, alkylamino-derivatized cyclodextrin, or succinyl-derivatized cyclodextrin.

Some exemplary mixed ether cyclodextrin derivatives.

**Table D.**

| Mixed ether CD derivative | Mixed ether CD derivative | Mixed ether CD derivative |
|---|---|---|
| Sulfobutyl-hydroxybutyl-CD (SBE-HBE-CD) | Sulfopropyl-hydroxybutyl-CD (SPE-HBE-CD) | Sulfoethyl-hydroxybutyl-CD (SEE-HBE-CD) |
| Sulfobutyl-hydroxypropyl-CD (SBE-HPE-CD) | Sulfopropyl-hydroxypropyl-CD (SPE-HPE-CD) | Sulfoethyl-hydroxypropyl-CD (SEE-HPE-CD) |
| Sulfobutyl-hydroxyethyl-CD (SBE-HEE-CD) | Sulfopropyl-hydroxyethyl-CD (SPE-HEE-CD) | Sulfoethyl-hydroxyethyl-CD (SEE-HEE-CD) |
| Sulfobutyl-hydroxybutenyl-CD (SBE-HBNE-CD) | Sulfopropyl-hydroxybutenyl-CD (SPE-HBNE-CD) | Sulfoethyl-hydroxybutenyl-CD (SEE-HBNE-CD) |
| Sulfobutyl-ethyl (SBE-EE-CD) | Sulfopropyl-ethyl (SPE-EE-CD) | Sulfoethyl-ethyl (SEE-EE-CD) |
| Sulfobutyl-methyl (SBE-ME-CD) | Sulfopropyl-methyl (SPE-ME-CD) | Sulfoethyl-methyl (SEE-ME-CD) |
| Sulfobutyl-propyl (SBE-PE-CD) | Sulfopropyl-propyl (SPE-PE-CD) | Sulfoethyl-propyl (SEE-PE-CD) |
| Sulfobutyl-butyl (SBE-BE-CD) | Sulfopropyl-butyl (SPE-BE-CD) | Sulfoethyl-butyl (SEE-BE-CD) |
| Sulfobutyl-carboxymethyl-CD (SBE-CME-CD) | Sulfopropyl-carboxymethyl-CD (SPE-CME-CD) | Sulfoethyl-carboxymethyl-CD (SEE-CME-CD) |
| Sulfobutyl-carboxyethyl-CD (SBE-CEE-CD) | Sulfopropyl-carboxyethyl-CD (SPE-CEE-CD) | Sulfoethyl-carboxyethyl-CD (SEE-CEE-CD) |
| Sulfobutyl-acetate-CD (SBE-AA-CD) | Sulfopropyl-acetate-CD (SPE-AA-CD) | Sulfoethyl-acetate-CD (SEE-AA-CD) |
| Sulfobutyl-propionate-CD (SBE-PA-CD) | Sulfopropyl-propionate-CD (SPE-PA-CD) | Sulfoethyl-propionate-CD (SEE-PA-CD) |
| Sulfobutyl-butyrate-CD (SBE-BA-CD) | Sulfopropyl-butyrate-CD (SPE-BA-CD) | Sulfoethyl-butyrate-CD (SEE-BA-CD) |
| Sulfobutyl-methoxycarbonyl-CD (SBE-MC-CD) | Sulfopropyl-methoxycarbonyl-CD (SPE-MC-CD) | Sulfoethyl-methoxycarbonyl-CD (SEE-MC-CD) |
| Sulfobutyl-ethoxycarbonyl-CD (SBE-EC-CD) | Sulfopropyl-ethoxycarbonyl-CD (SPE-EC-CD) | Sulfoethyl-ethoxycarbonyl-CD (SEE-EC-CD) |
| Sulfobutyl-propoxycarbonyl-CD (SBE-PC-CD) | Sulfopropyl-propoxycarbonyl-CD (SPE-PC-CD) | Sulfoethyl-propoxycarbonyl-CD (SEE-PC-CD) |
| Hydroxybutyl-hydroxybutenyl-CD (HBE-HBNE-CD) | Hydroxypropyl-hydroxybutenyl-CD (HPE-HBNE-CD) | Hydroxyethyl-hydroxybutenyl-CD (HEE-HBNE-CD) |
| Hydroxybutyl-ethyl (HBE-EE-CD) | Hydroxypropyl-ethyl (HPE-EE-CD) | Hydroxyethyl-ethyl (HEE-EE-CD) |
| Hydroxybutyl-methyl (HBE-ME-CD) | Hydroxypropyl-methyl (HPE-ME-CD) | Hydroxyethyl-methyl (HEE-ME-CD) |
| Hydroxybutyl-propyl (HBE-PE-CD) | Hydroxypropyl-propyl (HPE-PE-CD) | Hydroxyethyl-propyl (HEE-PE-CD) |
| Hydroxybutyl-butyl (HBE-BE-CD) | Hydroxypropyl-butyl (HPE-BE-CD) | Hydroxyethyl-butyl (HEE-BE-CD) |
| Hydroxybutyl-carboxymethyl-CD (HBE-CME-CD) | Hydroxypropyl-carboxymethyl-CD (HPE-CME-CD) | Hydroxyethyl-carboxymethyl-CD (HEE-CME-CD) |
| Hydroxybutyl-carboxyethyl-CD (HBE-CEE-CD) | Hydroxypropyl-carboxyethyl-CD (HPE-CEE-CD) | Hydroxyethyl-carboxyethyl-CD (HEE-CEE-CD) |
| Hydroxybutyl-acetate-CD (HBE-AA-CD) | Hydroxypropyl-acetate-CD (HPE-AA-CD) | Hydroxyethyl-acetate-CD (HEE-AA-CD) |
| Hydroxybutyl-propionate-CD (HBE-PA-CD) | Hydroxypropyl-propionate-CD (HPE-PA-CD) | Hydroxyethyl-propionate-CD (HEE-PA-CD) |
| Hydroxybutyl-butyrate-CD (HBE-BA-CD) | Hydroxypropyl-butyrate-CD (HPE-BA-CD) | Hydroxyethyl-butyrate-CD (HEE-BA-CD) |
| Hydroxybutyl-methoxycarbonyl-CD (HBE-MC-CD) | Hydroxypropyl-methoxycarbonyl-CD (HPE-MC-CD) | Hydroxyethyl-methoxycarbonyl-CD (HEE-MC-CD) |
| Hydroxybutyl-ethoxycarbonyl-CD (HBE-EC-CD) | Hydroxypropyl-ethoxycarbonyl-CD (HPE-EC-CD) | Hydroxyethyl-ethoxycarbonyl-CD (HEE-EC-CD) |
| Hydroxybutyl-propoxycarbonyl-CD (HBE-PC-CD) | Hydroxypropyl-propoxycarbonyl-CD (HPE-PC-CD) | Hydroxyethyl-propoxycarbonyl-CD (HEE-PC-CD) |
| Hydroxybutenyl-ethyl (HBNE-EE-CD) | Hydroxypropenyl-ethyl (HPNE-EE-CD) | Hydroxypentenyl-ethyl (HPTNE-EE-CD) |
| Hydroxybutenyl-methyl (HBNE-ME-CD) | Hydroxypropenyl-methyl (HPNE-ME-CD) | Hydroxypentenyl-methyl (HPTNE-ME-CD) |
| Hydroxybutenyl-propyl (HBNE-PE-CD) | Hydroxypropenyl-propyl (HPNE-PE-CD) | Hydroxypentenyl-propyl (HPTNE-PE-CD) |
| Hydroxybutenyl-butyl (HBNE-BE-CD) | Hydroxypropenyl-butyl (HPNE-BE-CD) | Hydroxypentenyl-butyl (HPTNE-BE-CD) |
| Hydroxybutenyl-carboxymethyl-CD (HBNE-CME-CD) | Hydroxypropenyl-carboxymethyl-CD (HPNE-CME-CD) | Hydroxypentenyl-carboxymethyl-CD (HPTNE-CME-CD) |
| Hydroxybutenyl-carboxyethyl-CD (HBNE-CEE-CD)- | Hydroxypropenyl-carboxyethyl-CD (HPNE-CEE-CD) | Hydroxypentenyl-carboxyethyl-CD (HPTNE-CEE-CD) |
| Hydroxybutenyl-acetate-CD (HBNE-AA-CD) | Hydroxypropenyl-acetate-CD (HPNE-AA-CD) | Hydroxypentenyl-acetate-CD (HPTNE-AA-CD) |
| Hydroxybutenyl-propionate-CD (HBNE-PA-CD) | Hydroxypropenyl-propionate-CD (HPNE-PA-CD) | Hydroxypentenyl-propionate-CD (HPTNE-PA-CD) |
| Hydroxybutenyl-butyrate-CD (HBNE-BA-CD) | Hydroxypropenyl-butyrate-CD (HPNE-BA-CD) | Hydroxypentenyl-butyrate-CD (HPTNE-BA-CD) |
| Hydroxybutenyl-methoxycarbonyl-CD (HBNE-MC-CD) | Hydroxypropenyl-methoxycarbonyl-CD (HPNE-MC-CD) | Hydroxypentenyl-methoxycarbonyl-CD (HPTNE-MC-CD) |
| Hydroxybutenyl-ethoxycarbonyl-CD (HBNE-EC-CD) | Hydroxypropenyl-ethoxycarbonyl-CD (HPNE-EC-CD) | Hydroxypentenyl-ethoxycarbonyl-CD (HPTNE-EC-CD) |
| Hydroxybutenyl-propoxycarbonyl-CD (HBNE-PC-CD) | Hydroxypropenyl-propoxycarbonyl-CD (HPNE-PC-CD) | Hydroxypentenyl-propoxycarbonyl-CD (HPTNE-PC-CD) |

The terms "sulfoalkyl ether cyclodextrin" and "SAE-CD" as used herein refers to a cyclodextrin derivative containing a sulfoalkyl ether substituent, such as a (C₂₋₆ alkylene)-SO₃⁻. The sulfoalkyl derivative of cyclodextrin can be a single derivative or a mixture of derivatives. Since the cyclodextrin derivatives contain sulfonyl groups, they can be charged species. The sulfoalkyl ether cyclodextrin can be either substituted at least at one of the primary hydroxyl groups of cyclodextrin or they are substituted at both the primary hydroxyl groups and at the 3-positioned hydroxyl group. Substitution at the 2-position is also possible. Examples of sulfoalkyl ether cyclodextrin include sulfobutyl ether β cyclodextrin.

In some embodiments, the cyclodextrin is a compound of Formula 1: 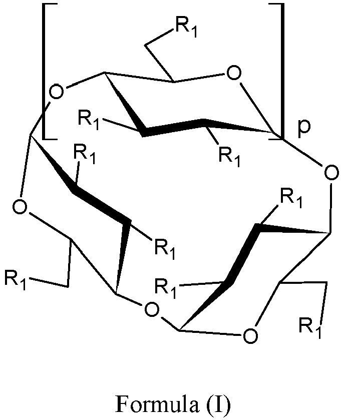 or a pharmaceutically acceptable salt thereof, wherein:
p is 4, 5, or 6, and each R₁ is selected from -OH, or -O-(C₁-C₈ alkylene)-SO₃T, and
each T is independently hydrogen or pharmaceutically acceptable cation,
provided that at least one R₁ is -OH.

In some embodiments, each R₁ is independently-OH or -O-(C₁-C₈ alkylene)-SO₃T, provided that at least one R₁ is OH and at least one R₁ is -O-(C₁-C₈ alkylene)-SO₃T, wherein T is a hydrogen or pharmaceutically acceptable cation. In some embodiments, at least one R₁ is independently -OH or -O-(C₁₋₄ alkylene)-SO₃T. In some embodiments, at least one R₁ is independently a -O-(CH₂)_{g}SO₃T group, wherein g is 2 to 6, or 2 to 4. In some embodiments, at least one R₁ is independently -OCH₂CH₂CH₂SO₃T or - OCH₂CH₂CH₂CH₂SO₃T. In some embodiments, at least one R₁ is -OCH₂CH₂CH₂-OH. In some embodiments, each R₁ is independently -OH or O-(C₁-C₆ alkyl)-OH, provided that at least one R₁ is O-(C₁-C₆ alkyl)-OH. In some embodiments, T is independently hydrogen or sodium. In some embodiments, T is H. In some embodiments, T is Na⁺. In some embodiments, each T is independently selected from an alkali metal, an alkaline earth metals, ammonium ions, and amine cations such as the, and combinations thereof. In some embodiments, each T is independently selected from Li⁺, Na⁺, K⁺, Ca⁺², Mg⁺², amine, and any combination thereof. In some embodiments, each T is independently an amine cation selected from (C₁-C₆)-alkylamines, piperidine, pyrazine, (C₁-C₆)-alkanolamine, ethylenediamine and (C₄-C₈)-cycloalkanolamine.

In some embodiments, each R₁ is independently -OH or -O-(C₁-C₈ alkyl), provided that at least one R₁ is OH and at least one R₁ is -O-(C₁-C₈ alkyl). In some embodiments, each R₁ is independently selected from methyl ether, ethyl ether, propyl ether, butyl ether, pentyl ethyl, and hexyl ether.

In some embodiments, each R₁ is independently -OH or O-(C₁-C₆ alkyl)-OH, provided that at least one R₁ is OH and at least one R₁ is O-(C₁-C₆ alkyl)-OH. In some embodiments, at least one R₁ is -O-(C₁-C₆ alkyl)-OH. In some embodiments, each R₁ is independently selected from hydroxymethyl ether, hydroxyethyl ether, hydroxypropyl ether, hydroxybutyl ether, hydroxypentyl ether, and hydroxyhexyl ether.

In some embodiments, p is 4. In some embodiments, p is 5. In some embodiments, p is 6.

In some embodiments, a cyclodextrin derivative such as a sulfoalkyl ether cyclodextrin, an alkylated cyclodextrin, or a hydroxyalkyl ether cyclodextrin can have an average degree of substitution (ADS) of 2 to 9, 4 to 8, 4 to 7.5, 4 to 7, 4 to 6.5, 4.5 to 8, 4.5 to 7.5, 4.5 to 7, 5 to 8, 5 to 7.5, 5 to 7, 5.5 to 8, 5.5 to 7.5, 5.5 to 7, 5.5 to 6.5, 6 to 8, 6 to 7.5, 6 to 7.1, 6.5 to 7.1, 6.2 to 6.9, or 6.5 per cyclodextrin, and the remaining substituents are -H.

Some embodiments provide compositions containing a single type of cyclodextrin derivative having the structure set out in formula (I), where the composition overall contains on the average at least 1 and up to 3n + 6 alkylsulfonic acid moieties per cyclodextrin molecule. The compositions described herein also includes compositions containing cyclodextrin derivatives having a narrow or wide range for degree of substitution and high or low degree of substitution. These combinations can be optimized as needed to provide cyclodextrins having particular properties.

Exemplary SAE-CD derivatives include SBE4-β-CD, SBE7-β-CD, SBE11-β-CD, SBE7-γ-CD and SBE5-γ-CD which correspond to SAE-CD derivatives of the formula I wherein p= 5, 5, 5, 6 and 6, respectively; and there are on average 4, 7, 11, 7 and 5 sulfoalkyl ether substituents present, respectively. Other exemplary SAE-CD derivatives include those of the formula SAEx-R-CD (Formula 2), wherein SAE is sulfomethyl ether (SME), sulfoethyl ether (SEE), sulfopropyl ether (SPE), sulfobutyl ether (SBE), sulfopentyl ether (SPtE), or sulfohexyl ether (SHE); x (average or specific degree of substitution) is 1-18, 1-21, or 1-24; R (ring structure of parent cyclodextrin) is α, β or γ, respectively; and CD is cyclodextrin. The SAE functional group includes a cationic counterion as disclosed herein or generally as used in the pharmaceutical industry for the counterion of any acidic group. Since SAE-CD is a poly-anionic cyclodextrin, it can be provided in different salt forms. Suitable counterions for the SAE functional group(s) include cationic organic atoms or molecules and cationic inorganic atoms or molecules. The SAE-CD can include a single type of counterion or a mixture of different counterions. The properties of the SAE-CD can be modified by changing the identity of the counterion present. For example, a first salt form of SAE-CD can have a greater electrostatic charge than a different second salt form of SAE-CD. The calcium salt form has been found to be more electronegative than the sodium salt form. Likewise, a SAE-CD having a first degree of substitution can have a greater electrostatic charge than a second SAE-CD having a different degree of substitution.

Some embodiments provide compositions of SAE-CD where the SAE-CD is a sulfobutyl ether derivative of beta cyclodextrin (SBE-β-CD) having the following structure: 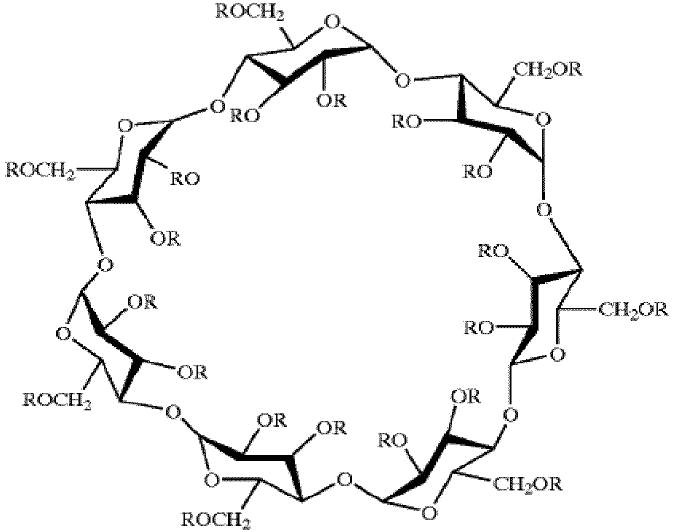 wherein each R is independently -H or -(CH₂)₄-SO₃Na and the average degree of substitution with -(CH₂)₄-SO₃Na groups is between 6 and 7.1.

Methods of preparing SAE-CD derivatives are varied but generally include the general steps of sulfoalkylation followed by isolation. The chemical property profile of the SAE-CD is established during the sulfoalkylation step. For example, altering reaction conditions during sulfoalkylation can vary the average degree of substitution for and the average regiochemical distribution of sulfoalkyl groups in the SAE-CD. The alkyl chain length of the sulfoalkyl functional group is determined according the sulfoalkylating agent used. And use of a particular alkalizing agent during alkylation would result in formation of a particular SAE-CD salt, unless an ion exchange step were performed subsequent to sulfoalkylation.

In general, known processes for the sulfoalkylation step include, for example: 1) exposure of underivatized parent cyclodextrin under alkaline conditions to an alkylating agent, e.g. alkyl sultone or a haloalkylsulfonate; 2) optional addition of further alkalizing agent to the reaction milieu to consume excess alkylating agent; and 3) neutralization of the reaction medium with acidifying agent. The vast majority of literature processes conduct the sulfoalkylation step in aqueous media; however, some references disclose the use of pyridine, dioxane, or DMSO as the reaction solvent for sulfoalkylation. Literature discloses the use of an alkalizing agent in order to accelerate the sulfoalkylation reaction.

Upon completion of the sulfoalkylation step, isolation and purification of the SAE-CD is conducted.

Several different isolation processes for SAE-CD following sulfoalkylation and neutralization are described. In general, an aqueous liquid containing SAE-CD is dried to remove water to form a solid. The literature suggests various methods for removal of water from an aqueous solution containing SAE-CD. Such methods include conventional freeze-drying, spray drying, oven drying, vacuum oven drying, roto-evaporation under reduced pressure, vacuum drying or vacuum drum drying. See, for example, Ma (S.T.P. Pharma. Sciences (1999), 9(3), 261-266), CAPTISOL^{®} (sulfobutyl ether beta-cyclodextrin sodium; Pharmaceutical Excipients 2004; Eds. R. C. Rowe, P. J. Sheskey, S. C. Owen; Pharmaceutical Press and American Pharmaceutical Association, 2004) and other references regarding the preparation of SAE-CD derivatives.

Suitable methods for preparing a SAE-CD- raw material for use in preparing the SAE-CD composition for use as described herein are disclosed in U.S. Patents No. 5,376,645, No. 5,874,418, and No. 5,134,127 to Stella et al.; U.S. Patent No. 3,426,011 to Parmerter et al.; Lammers et al. (Reel. Trav. CMm. Pays-Bas (1972), 91(6), 733-742); Staerke (1971), 23(5), 167-171); Qu et al. (J Inclusion Phenom. Macro. Chem., (2002), 43, 213-221); U.S. Patent No. 5,241,059 to Yoshinaga; U.S Patent No. 6,153,746 to Shah; PCT International Publication No. WO 2005/042584 to Stella et al; Adam et al. (J. Med. Chem. (2002), 45, 1806-1816); PCT International Publication No. WO 01/40316 to Zhang et al.; Tarver et al. (Bioorganic & Medicinal Chemistry (2002), 10, 1819-1827); Ma (S.T.P. Pharma. Sciences (1999), 9(3), 261-266); Jung et al. (J Chromat. 1996, 755, 81- 88); and Luna et al. (Carbohydr. Res. 1997, 299, 103-110).

The SAE-CD raw material can be included in the liquid feed used in the fluidized bed spray drying process as described in US Patent No. 8,049,003. Other methods for removal of water from an aqueous solution containing SAE-CD can include conventional freeze-drying, spray drying, oven drying, vacuum oven drying, roto-evaporation under reduced pressure, vacuum drying or vacuum drum drying. See, for example, Ma (S.T.P. Pharma. Sciences (1999), 9(3), 261-266), CAPTISOL^{®}(sulfobutyl ether beta-cyclodextrin sodium; Pharmaceutical Excipients 2004; Eds. R. C. Rowe, P. J. Sheskey, S. C. Owen; Pharmaceutical Press and American Pharmaceutical Association, 2004) and other references regarding the preparation of SAE-CD derivatives.

The SAE-CD composition described herein can also include a combination of derivatized cyclodextrin (SAE-CD) and underivatized cyclodextrin. For example, a SAE-CD composition can be made to include underivatized cyclodextrin in the amount of 0 to less than 50% by wt. of the total cyclodextrin present. Exemplary embodiments of the SAE-CD composition include those comprising 0-5% by wt., 5-50% by wt., less than 5%, less than 10%, less than 20%, less than 30%, less than 40%, or less than 50% underivatized cyclodextrin.

### Compositions Containing Busulfan and Cyclodextrin

Some embodiments relate to a pharmaceutical composition including a clear aqueous solution that comprises busulfan; and cyclodextrin.

In some embodiments, the busulfan concentration is greater than about 4 mg/ml. In some embodiments, the busulfan concentration is about 0.5 mg/ml. In some embodiments, the busulfan concentration is about 0.55 mg/ml. In some embodiments, the busulfan concentration is greater than about 0.1, 0.2, 0.3, 0.4, 0.5, 0.6, 0.7, 0.8, 0.9, 1, 1.5, 2, 2.5, 3, 3.5, 4, 4.5, 5, 5.6, 6, 6.5, 7, 7.5, or 8 mg/ml. In some embodiments, the busulfan concentration is less than about 10, 9, 8, 7, 6, 5, 4, 3.5, 3, 2.5, 2, 1.5, 1, 0.9, 0.8, 0.7, 0.6, 0.5, 0.4, 0.3, or 0.2 mg/ml. In some embodiments, the busulfan concentration may range from any lower limit of about 0.1, 0.2, 0.3, 0.4, 0.5, 0.6, 0.7, 0.8, 0.9, 1, 1.5, 2, 2.5, 3, 3.5, 4, 4.5, 5, 5.6, 6, 6.5, 7, 7.5, or 8 mg/ml to any upper limit of about 10, 9, 8, 7, 6, 5, 4, 3.5, 3, 2.5, 2, 1.5, 1, 0.9, 0.8, 0.7, 0.6, 0.5, 0.4, 0.3, or 0.2 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the busulfan concentration can be in the range of about 1-10 mg/ml, about 2-8 mg/ml, about 3-6 mg/ml, about 0.2-4 mg/ml, about 0.2-3 mg/ml, about 0.3-3 mg/ml, about 0.5-2 mg/ml, or about 0.4-1 mg/ml.

In some embodiments, the molar ratio of cyclodextrin to busulfan is less than about 25, 22, 20, 19, 18, 17, 16, 15, 14, 13, 12, 11, 10, 9, 8, 7, 6, 5, 4, 3, 2, or 1. In some embodiments, the molar ratio of cyclodextrin to busulfan is less than about 9.1. In some embodiments, the molar ratio of cyclodextrin to busulfan is less than about 9. In some embodiments, the molar ratio of cyclodextrin to busulfan is less than about 8. In some embodiments, the molar ratio of cyclodextrin to busulfan is less than about 7. In some embodiments, the molar ratio of cyclodextrin to busulfan is less than about 6. In some embodiments, the molar ratio of cyclodextrin to busulfan is less than about 5. In some embodiments, the molar ratio of cyclodextrin to busulfan is about 3.1. In some embodiments, the molar ratio of cyclodextrin to busulfan is greater than about 2, 3, 4, 5, or 6. In some embodiments, the molar ratio of cyclodextrin to busulfan is greater than about 3. In some embodiments, the molar ratio of cyclodextrin to busulfan is in the range of about 3 to about 5.2. In some embodiments, the molar ratio of cyclodextrin to busulfan may range from any lower limit of about 2, 3, 4, 5, or 6 to any upper limit of about 25, 22, 20, 19, 18, 17, 16, 15, 14, 13, 12, 11, 10, 9, 8, 7, 6, 5, 4, 3, 2, or 1 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the molar ratio of cyclodextrin to busulfan is in the range of about 3 to about 10, about 3 to about 9, about 3 to about 7, or about 3 to about 5.

In some embodiments, the mass ratio of cyclodextrin to busulfan is about 42.5. In some embodiments, the mass ratio of cyclodextrin to busulfan is about 67.5. In some embodiments, the mass ratio of cyclodextrin to busulfan is about 80. In some embodiments, the mass ratio of cyclodextrin to busulfan is less than about 80, 70, 67.5, 65, 60, 55, 50, 45, 42.5, or 40. In some embodiments, the mass ratio of cyclodextrin to busulfan may range from any lower limit of about 10, 20, 25, 30, 35, 40, 45, 50, 55, 60, 65, 70, or 80 to any upper limit of about 90, 80, 70, 67.5, 65, 60, 55, 50, 45, 42.5, or 40 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the mass ratio of cyclodextrin to busulfan is in the range of about 40 to about 80, about 40 to about 60, or about 32 to about 54.

Some embodiments relate to a pharmaceutical composition, comprising a clear aqueous solution that includes busulfan; and sulfoalkyl ether cyclodextrin.

In some embodiments, the molar ratio of sulfoalkyl ether cyclodextrin to busulfan is in the range of about 3 to about 12. In some embodiments, the molar ratio of sulfoalkyl ether cyclodextrin to busulfan is less than 9.1. In some embodiments, the molar ratio of sulfoalkyl ether cyclodextrin to busulfan is less than about 9. In some embodiments, the molar ratio of sulfoalkyl ether cyclodextrin to busulfan is less than about 8. In some embodiments, the molar ratio of sulfoalkyl ether cyclodextrin to busulfan is less than about 7. In some embodiments, the molar ratio of sulfoalkyl ether cyclodextrin to busulfan is less than about 6. In some embodiments, the molar ratio of sulfoalkyl ether cyclodextrin to busulfan is less than about 5. In some embodiments, the molar ratio of sulfoalkyl ether cyclodextrin to busulfan is less than about 5.3. In some embodiments, the molar ratio of sulfoalkyl ether cyclodextrin to busulfan is about 3.1. In some embodiments, the molar ratio of sulfoalkyl ether cyclodextrin to busulfan is less than about 25, 22, 20, 19, 18, 17, 16, 15, 14, 13, 12, 11, 10, 9, 8, 7, 6, 5, 4, 3, 2, or 1. In some embodiments, the molar ratio of sulfoalkyl ether cyclodextrin to busulfan is greater than about 2, 3, 4, 5, or 6. In some embodiments, the molar ratio of sulfoalkyl ether cyclodextrin to busulfan is greater than about 3. In some embodiments, the molar ratio of sulfoalkyl ether cyclodextrin to busulfan is in the range of about 3 to about 5.2. In some embodiments, the molar ratio of sulfoalkyl ether cyclodextrin to busulfan may range from any lower limit of about 2, 3, 4, 5, or 6 to any upper limit of about 25, 22, 20, 19, 18, 17, 16, 15, 14, 13, 12, 11, 10, 9, 8, 7, 6, 5, 4, 3, 2, or 1 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the molar ratio of sulfoalkyl ether cyclodextrin to busulfan is in the range of about 3 to about 10, about 3 to about 9, about 3 to about 7, or about 3 to about 5.

Some embodiments relate to a pharmaceutical composition including a solid that comprises busulfan; and cyclodextrin; wherein at least about 25% of the busulfan in the composition is complexed with the sulfoalkyl ether cyclodextrin. In some embodiments, the solid is water soluble.

In some embodiments, a major portion of the busulfan in the composition is complexed with the sulfoalkyl ether cyclodextrin. In some embodiments, at least about 5%, 10%, 15%, 20%, 25%, 30%, 35%, 40%, 45%, 50%, 55%, 60%, 65%, 70%, 75%, 80%, 85%, 90%, 95%, or 99% of the busulfan in the composition is complexed with the sulfoalkyl ether cyclodextrin. In some embodiments, less than 100%, 99%, 95%, 90%, 85%, 80%, 75%, 70%, 65%, 60%, 55%, 50%, 45%, 40%, 35%, 30%, 25%, 20%, 15%, 10%, or 5% of the busulfan in the composition is complexed with the sulfoalkyl ether cyclodextrin. In some embodiments, the amount of the busulfan in the composition that is complexed with the sulfoalkyl ether cyclodextrin may range from any lower limit of 5%, 10%, 15%, 20%, 25%, 30%, 35%, 40%, 45%, 50%, 55%, 60%, 65%, 70%, 75%, 80%, 85%, 90%, 95%, or 99%, to any upper limit of 100%, 99%, 95%, 90%, 85%, 80%, 75%, 70%, 65%, 60%, 55%, 50%, 45%, 40%, 35%, 30%, 25%, or 20%, and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the amount of busulfan that is complexed with the cyclodextrin is in the range of about 20% to about 95%, about 50% to about 99%, about 80% to about 99%, about 90% to about 99%, or about 60% to about 100%.

In some embodiments, the mole fraction ratio of sulfoalkyl ether cyclodextrin to busulfan is in the range of about 3 to about 12. In some embodiments, the mole fraction ratio of sulfoalkyl ether cyclodextrin to busulfan is in the range of about 3 to about 10. In some embodiments, the mole fraction ratio of sulfoalkyl ether cyclodextrin to busulfan is less than 12. In some embodiments, the mole fraction ratio of sulfoalkyl ether cyclodextrin to busulfan is less than 9.1. In some embodiments, the mole fraction ratio of sulfoalkyl ether cyclodextrin to busulfan is less than 9. In some embodiments, the mole fraction ratio of sulfoalkyl ether cyclodextrin to busulfan is less than about 8. In some embodiments, the mole fraction ratio of sulfoalkyl ether cyclodextrin to busulfan is less than about 7. In some embodiments, the mole fraction ratio of sulfoalkyl ether cyclodextrin to busulfan is less than about 6. In some embodiments, the mole fraction ratio of sulfoalkyl ether cyclodextrin to busulfan is less than about 5. In some embodiments, the mole fraction ratio of sulfoalkyl ether cyclodextrin to busulfan is less than about 5.3. In some embodiments, the mole fraction ratio of sulfoalkyl ether cyclodextrin to busulfan is about 3.1. In some embodiments, the mole fraction ratio of sulfoalkyl ether cyclodextrin to busulfan is less than about 25, 22, 20, 19, 18, 17, 16, 15, 14, 13, 12, 11, 10, 9, 8, 7, 6, 5, 4, 3, 2, or 1. In some embodiments, the mole fraction ratio of sulfoalkyl ether cyclodextrin to busulfan is greater than about 2, 3, 4, 5, or 6. In some embodiments, the mole fraction ratio of sulfoalkyl ether cyclodextrin to busulfan may range from any lower limit of about 2, 3, 4, 5, or 6 to any upper limit of about 25, 22, 20, 19, 18, 17, 16, 15, 14, 13, 12, 11, 10, 9, 8, 7, 6, 5, 4, 3, 2, or 1 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the mole fraction ratio of sulfoalkyl ether cyclodextrin to busulfan is greater than about 3. In some embodiments, the mole fraction ratio of sulfoalkyl ether cyclodextrin to busulfan is in the range of about 3 to about 5.2. In some embodiments, the mole fraction ratio of sulfoalkyl ether cyclodextrin to busulfan is in the range of about 3 to about 10, about 3 to about 9, about 3 to about 7, or about 3 to about 5.

Some embodiments relate to a reconstituted solution obtained by adding a pharmaceutically acceptable solvent to the composition described herein, wherein the busulfan concentration is in the range of about 0.3 mg/ml to about 3 mg/ml.

In some embodiments, the mass ratio of busulfan to cyclodextrin is greater than about 0.01.

In some embodiments, the mass ratio of busulfan to cyclodextrin is greater than about 0.005, 0.01, 0.02, 0.03, 0.04, 0.05, 0.06, 0.07, 0.08, 0.09, 0.1, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.34, 0.35, 0.36, 0.37, 0.38, 0.39, 0.40, 0.45, 0.48, or 0.50. In some embodiments, the mass ratio of busulfan to cyclodextrin is less than about 5, 4, 3, 2, 1, 0.9, 0.8, 0.7, 0.6, 0.5, 0.4, 0.3, 0.2, 0.1, 0.05, 0.025, or 0.01. In some embodiments, the mass ratio of busulfan to cyclodextrin may range from any lower limit of about 0.005, 0.01, 0.02, 0.03, 0.04, 0.05, 0.06, 0.07, 0.08, 0.09, 0.1, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.34, 0.35, 0.36, 0.37, 0.38, 0.39, 0.40, 0.45, 0.48, or 0.50 to any upper limit of about 5, 4, 3, 2, 1, 0.9, 0.8, 0.7, 0.6, 0.5, 0.4, 0.3, 0.2, 0.1, 0.05, 0.025, or 0.01 and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the mass ratio of busulfan to cyclodextrin is in the range of about 0.01 to about 5, about 0.1 to about 2, or about 0.5 to about 1.

Some embodiments relate to a sterilized container comprising the composition described herein.

In some embodiments, the composition has a moisture content of less than 20%. In some embodiments, the composition has a moisture content of less than 50%, 40%, 30%, 25%, 20%, 10% or 5%. In some embodiments, the composition has a moisture content of greater than 0.1%, 1%, 5%, or 10%. In some embodiments, the composition has a moisture content in the range of about 0.1% to about 50%, 1% to about 30%, or about 5% to about 25%.

The composition described herein can have an osmolarity suitable for the parenteral injection. In some embodiments, the osmolality of the composition may range from any lower limit of about 100, 110, 120, 130, 140, 150, 160, 170, 180, 190, 200, 210, 220, 230, 240, 250, 260, 270, 280, 290, 300, 320, 340, 360, 380, or 400 mOsm to any upper limit of about 600, 500, 480, 460, 440, 420, 400, 390, 380, 370, 360, 350, 340, 330, 320, 310, 300, 290, 280, 270, 260, 250, 240, 230, 220, 210, 200, 180, 160, 140, 120, or 100 mOsm and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the composition has an osmolarity in the range of about 150 mOsm to about 600 mOsm, about 200 mOsmol/L to about 400 mOsm, about 200 to about 320 mOsm, or 285 to about 310 mOsm. In more specific embodiments, the composition can have an osmolarity in the range of about 221 to about 280 mOsm. In one embodiment, the osmolarity is about 270 mOsm. In some embodiments, the composition can have an osmolarity close to human plasma osmolarity. In some embodiments, the osmolarity of the composition is greater than 100, 110, 120, 130, 140, 150, 160, 170, 180, 190, 200, 210, 220, 230, 240, 250, 260, 270, 280, 290, 300, 320, 340, 360, 380, or 400 mOsm. In some embodiments, the osmolarity of the composition is less than 600, 500, 480, 460, 440, 420, 400, 390, 380, 370, 360, 350, 340, 330, 320, 310, 300, 290, 280, 270, 260, 250, 240, 230, 220, 210, 200, 180, 160, 140, 120, or 100 mOsm.

In some embodiments, the cyclodextrin is a sulfubutyl ether cyclodextrin. In some embodiments, the cyclodextrin is hydroxy propyl beta cyclodextrin. In some embodiments, the cyclodextrin is an alpha cyclodextrin. In some embodiments, the cyclodextrin is a sulfubutyl butyl ether alpha cyclodextrin. In some embodiments, the cyclodextrin is a sulfubutyl butyl ether gamma cyclodextrin. In some embodiments, the cyclodextrin is a sulfubutyl ether beta cyclodextrin having a ADS of about 2. In some embodiments, the cyclodextrin is a sulfubutyl ether beta cyclodextrin having a ADS of about 6.5.

In some embodiments the composition described herein includes less than 30% (w/w) of dimethylacetamide. In some embodiments, the composition includes less than about 90%, 80%, 70%, 60%, 50%, 45%, 40%, 35%, 30%, 25%, 20%, 10%, 9%, 8%, 7%, 6%, 5%, 4%, 3%, 2%, 1%, 0.5%, 0.25%, 0.1%, or 0.05% (w/w) of dimethylacetamide. In some embodiments, the dimethylacetamide in the composition may range from any lower limit of about 0%, 0.0001%, 0.001%, or 0.01% to any upper limit of about 90%, 80%, 70%, 60%, 50%, 45%, 40%, 35%, 30%, 25%, 20%, 10%, 9%, 8%, 7%, 6%, 5%, 4%, 3%, 2%, 1%, 0.5%, 0.25%, 0.1%, 0.05% (w/w) and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the dimethylacetamide in the composition may be in the range of about 0% to about 1%, about 0.0001% to about 1%, or 0.001% to about 0.05%.

In some embodiments, the pharmaceutical composition described herein does not comprise dimethylacetamide. In some embodiments, the pharmaceutical composition described herein has substantially no dimethylacetamide. In some embodiments, the amount of dimethylacetamide in the pharmaceutical composition described herein is less than about 10%, 8%, 5%, 4%, 3%, 2%, 1%, 0.8%, 0.6%, 0.5%, 0.3%, 0.1%, 0.05%, 0.02%, 0.01%, 0.005%, or 0.001% by weight, based on the total weight of the composition.

**In** some embodiments, the composition described herein does not comprise polyethylene glycol (PEG) (*e.g.,* PEG 400). In some embodiments, the pharmaceutical composition described herein has substantially no PEG (*e.g.,* PEG 400). In some embodiments, the amount of PEG (*e.g.,* PEG 400) in the pharmaceutical composition described herein is less than about 10%, 8%, 5%, 4%, 3%, 2%, 1%, 0.8%, 0.6%, 0.5%, 0.3%, 0.1%, 0.05%, 0.02%, 0.01%, 0.005%, or 0.01% by weight, based on the total weight of the composition.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

The compositions described herein are preferably provided in unit dosage form. As used herein, a "unit dosage form" is a composition containing an amount of a compound that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, though a single administration is not specifically excluded. The skilled artisan will recognize that the formulation does not specifically contemplate the entire course of therapy and such decisions are left for those skilled in the art of treatment rather than formulation.

The compositions useful as described above may be in any of a variety of suitable forms for a variety of routes for administration, for example, for oral, nasal, rectal, topical (including transdermal), ocular, intracerebral, intracranial, intrathecal, intra-arterial, intravenous, intramuscular, or other parental routes of administration. In some embodiments, the composition described herein can be made into in the form of a solid (e.g., lyophilized powder) that can be reconstituted with a suitable liquid prior to administration. In some embodiments, the composition described herein can be in a liquid form ready for administration. The skilled artisan will appreciate that oral and nasal compositions include compositions that are administered by inhalation, and made using available methodologies. In some embodiments, the composition described herein can be administered through any suitable infusion devices, portable devices, or wearable devices. Depending upon the particular route of administration desired, a variety of pharmaceutically-acceptable carriers well-known in the art may be used. Pharmaceutically-acceptable carriers include, for example, liquid fillers, diluents, hydrotropies, surface-active agents, and encapsulating substances. Optional pharmaceutically-active materials may be included, which do not substantially interfere with the inhibitory activity of the compound acetaminophen. The amount of carrier employed in conjunction with the compound is sufficient to provide a practical quantity of material for administration per unit dose of the compound. Techniques and compositions for making dosage forms useful in the methods described herein are described in the following references: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004).

Various oral dosage forms can be used, including liquid forms. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carriers suitable for the preparation of unit dosage forms for peroral administration is well-known in the art. Peroral compositions include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Compositions described herein may optionally include other drug actives.

A liquid composition, which is formulated for topical ophthalmic use, is formulated such that it can be administered topically to the eye. The comfort may be maximized as much as possible, although sometimes formulation considerations (e.g. drug stability) may necessitate less than optimal comfort. In the case that comfort cannot be maximized, the liquid may be formulated such that the liquid is tolerable to the patient for topical ophthalmic use. Additionally, an ophthalmically acceptable liquid may either be packaged for single use, or contain a preservative to prevent contamination over multiple uses.

For ophthalmic application, solutions or medicaments are often prepared using a physiological saline solution as a major vehicle. Ophthalmic solutions may preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric, acetate and phenylmercuric nitrate. A useful surfactant is, for example, Tween 80. Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

Ophthalmically acceptable antioxidants include, but are not limited to, sodium metabisulfite, sodium thiosulfate, acetylcysteine, butylated hydroxyanisole and butylated hydroxytoluene.

Other excipient components, which may be included in the ophthalmic preparations, are chelating agents. A useful chelating agent is edetate disodium, although other chelating agents may also be used in place or in conjunction with it.

For topical use, creams, ointments, gels, solutions or suspensions, etc., containing the composition disclosed herein are employed. Topical formulations may generally be comprised of a pharmaceutical carrier, co-solvent, emulsifier, penetration enhancer, preservative system, and emollient.

For intravenous or intramuscular administration, the compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. Accordingly, buffers including acetate buffers, citrate buffers, phosphate buffers and borate buffers may be used to adjust the pH of these formulations as needed. Antioxidant excipients may include sodium bisulfite, acetone sodium bisulfite, sodium formaldehyde, sulfoxylate, thiourea, and edetate disodium. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The compositions for intravenous or intramuscular administration may be provided to caregivers in the form of one more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a composition described herein and another agent, the combination may be provided to caregivers as a mixture, or the caregivers may mix the two agents prior to administration, or the two agents may be administered separately.

The actual dose of the active compositions described herein depends on the specific composition, and on the condition to be treated; the selection of the appropriate dose is well within the knowledge of the skilled artisan. In some embodiments, a daily dose may be from about 0.25 mg/kg to about 120 mg/kg or more of body weight, from about 0.5 mg/kg or less to about 100 mg/kg, from about 1.0 mg/kg to about 80 mg/kg of body weight, or from about 1.5 mg/kg to about 75 mg/kg of body weight. Thus, for administration to a 70 kg person, the dosage range would be from about 17 mg per day to about 8000 mg per day, from about 35 mg per day or less to about 7000 mg per day or more, from about 70 mg per day to about 6000 mg per day, from about 100 mg per day to about 5000 mg per day, or from about 200 mg to about 3000 mg per day.

In some embodiments, the composition described herein can be administered as a 15-minute intravenous infusion. In some embodiments, the composition described herein can be administered as a 5 min to about 30 min intravenous infusion. The composition described herein can be given as a single or repeated dose.

In some embodiments, the composition can be administered through subcutaneous infusion. In some embodiments, the composition described herein can be administered through subcutaneous infusion in combination with Hylenex^{®} recombinant.

In some embodiments, for adult and adolescent patients weighing 50 kg and over, the dosage for the active compositions described herein can be 1000 mg every 6 hours or 650 mg every 4 hours to a maximum of 4000 mg per day. In some embodiments, for adult and adolescent patients weighing 50 kg and over, the dosage for the active compositions described herein can be in the range of about 500 mg to 1500 mg every 6 hours or in the range of about 300 mg to about 1000 mg every 4 hours. In some embodiments, for adult and adolescent patients weighing 50 kg and over, the maximum dosage per day can be in the range of about 2000 mg to about 6000 mg.

In some embodiments, for adult and adolescent patients weighing under 50 kg, the dosage for the active compositions described herein can be 15 mg/kg every 6 hours or 12.5 mg/kg every 4 hours to a maximum of 75 mg/kg per day. In some embodiments, for adult and adolescent patients weighing under 50 kg, the dosage for the active compositions described herein can be in the range of about 10 mg/kg to about 20 mg/kg every 6 hours or 8 mg/kg to about 15 mg/kg every 4 hours. In some embodiments, for adult and adolescent patients weighing under 50 kg, the maximum dosage per day can be in the range of about 50 mg/kg to about 100 mg/kg.

In some embodiments, for children 2 years to 12 years old, the dosage for the active compositions described herein can be 15 mg/kg every 6 hours or 12.5 mg/kg every 4 hours to a maximum of 75 mg/kg per day. In some embodiments, for children 2 years to 12 years old, the dosage for the active compositions described herein can be in the range of about 10 mg/kg to about 20 mg/kg every 6 hours or 8 mg/kg to about 15 mg/kg every 4 hours. In some embodiments, for children 2 years to 12 years old, the maximum dosage per day can be in the range of about 50 mg/kg to about 100 mg/kg.

In some embodiments, the minimum dosing interval can be 4 hours. In some embodiments, the minimum dosing interval can be in the range of about 1 hour to 8 hours. In some embodiments, the minimum dosing interval can be 1, 2, 3, 4, 5, 6, 7, or 8 hours.

### Method of Preparation

Some embodiments relate to a process for preparing a busulfan composition, comprising:
combining busulfan and an organic solvent to provide a clear solution;
combining the clear solution with a cyclodextrin to provide a first mixture;
removing the solvent from the first mixture to provide a second mixture; and
freeze-drying the second mixture to provide the busulfan composition.

Various solvents can be used to initially dissolve busulfan. In some embodiments, the organic solvent is selected from dimethylacetamide, polyethylene glycol, acetone, and any combination thereof. In some embodiments, the solvent is acetone. In some embodiments, the solvent is dimethylacetamide. In some embodiments, the solvent is dimethyl sulfoxide. In some embodiments, the solvent is polyethylene glycol. In some embodiments, the solvent is dimethylacetamide and polyethylene glycol. In some embodiments, the organic solvent is not dimethylacetamide. In some embodiments, the organic solvent is not polyethylene glycol.

Some embodiments relate to a process for preparing a busulfan composition, comprising:
combining busulfan and an organic solvent to provide a clear solution, wherein the organic solvent is selected from dimethylacetamide, acetone, and any combination thereof;
combining the clear solution with a cyclodextrin to provide a first mixture; and
drying the first mixture to provide the busulfan composition.

Some embodiments relate to a process for preparing a busulfan composition, comprising:
combining busulfan and acetone to provide a clear solution;
combining the clear solution with a cyclodextrin to provide a first mixture; and
drying the first mixture to provide the busulfan composition.

In some embodiments, removing the organic solvent comprises removing the organic solvent by rotary evaporator. In some embodiments, drying the first mixture further comprises lyophilizing. In some embodiments, drying the first mixture further comprises freeze-drying.

In some embodiments, combining the cyclodextrin with the clear busulfan solution comprises combining the clear busulfan solution with a cyclodextrin solution. In some embodiments, the cyclodextrin solution is added to the clear busulfan solution. In some embodiments, the cyclodextrin solution is an aqueous cyclodextrin solution.

In some embodiments, the process described herein includes combining the busulfan composition with a parenterally acceptable solvent to form a busulfan concentrate.

In some embodiments, the process described herein includes diluting the busulfan concentrate with a pharmaceutically acceptable diluent.

In some embodiments, the parenterally acceptable solvent is selected from water, saline, cyclodextrin solution, and any combination thereof. In some embodiments, the parenterally acceptable solvent is selected from water, saline, cyclodextrin solution, and any combination thereof. In some embodiments, the parenterally acceptable solvent is sugar solution, such as lactose, glucose and sucrose; starches, such as corn starch and potato starch; pyrogen-free water; isotonic saline; and phosphate buffer solutions. In some embodiments, the parenterally acceptable solvent is a cyclodextrin solution, and the cyclodextrin can be any cyclodextrin described herein, including any of the cyclodextrin or cyclodextrin derivatives listed in Tables A to D.

In some embodiments, the pharmaceutically acceptable diluent is selected from water, saline, cyclodextrin solution, and any combination thereof. In some embodiments, the pharmaceutically acceptable diluent is selected from water, saline, cyclodextrin solution, and any combination thereof. In some embodiments, the pharmaceutically acceptable diluent is sugar solution, such as lactose, glucose and sucrose; starches, such as corn starch and potato starch; pyrogen-free water; isotonic saline; and phosphate buffer solutions. In some embodiments, the pharmaceutically acceptable pharmaceutically acceptable diluent is a cyclodextrin solution, and the cyclodextrin can be any cyclodextrin described herein, including any of the cyclodextrin or cyclodextrin derivatives listed in Tables A to D.

In some embodiments, the second-mixture is a clear solution.

In some embodiments, the parenterally acceptable solvent is a cyclodextrin solution. In some embodiments, the parenterally acceptable solvent is a sulfobutyl ether cyclodextrin solution. In some embodiments, the cyclodextrin solution has a concentration greater than about 10, 20, 30, 40, 50, 60, 70, 80, 90, 100, 110, 120, 130, 140, 150, 160, 170, 180, 190, or 200 mg/ml. In some embodiments, the cyclodextrin solution has a concentration less than about 50, 60, 70, 80, 90, 100, 110, 120, 130, 140, 150, 160, 170, 180, 190, 200, 210, 220, 230, 240, 250, 260, 270, 280, 290, or 300 mg/ml. In some embodiments, cyclodextrin (e.g., sulfobutyl ether cyclodextrin) solution has a concentration that may range from any lower limit of about 10, 20, 30, 40, 50, 60, 70, 80, 90, 100, 110, 120, 130, 140, 150, 160, 170, 180, 190, or 200 mg/ml to any upper limit of about 50, 60, 70, 80, 90, 100, 110, 120, 130, 140, 150, 160, 170, 180, 190, 200, 210, 220, 230, 240, 250, 260, 270, 280, 290, or 300 mg/ml and encompass any subset between the upper and lower limits. Some of the lower limits listed above are greater than some of the listed upper limits, one skilled in the art will recognize that the selected subset will require the selection of an upper limit in excess of the selected lower limit. For example, in some embodiments, the cyclodextrin (e.g., sulfobutyl ether cyclodextrin) solution has a concentration in the range of about 10 to 500 mg/ml, about 50 to about 300 mg/ml, about 80 to about 200 mg/ml, or about 100 to150 mg/ml.

Some embodiments relate to a process of combining busulfan and sulfoalkyl ether cyclodextrin.

### Method of Treatment

Some embodiments relate to a pharmaceutical composition described herein for use in a method of treatment, including reconstituting the pharmaceutical composition described herein; and administering the reconstituted pharmaceutical composition to a subject in need thereof.

In some embodiments, the reconstituting comprises adding a parenterally acceptable solvent to the pharmaceutical composition. In some embodiments, the reconstituting comprises adding a pharmaceutically acceptable diluent to the pharmaceutical composition. In some embodiments, the parenterally acceptable solvent or pharmaceutically acceptable diluent is selected from water, saline, cyclodextrin solution, and any combination thereof. In some embodiments, the parenterally acceptable solvent or pharmaceutically acceptable diluent is sugar solution, such as lactose, glucose and sucrose; starches, such as corn starch and potato starch; pyrogen-free water; isotonic saline; and phosphate buffer solutions. In some embodiments, the parenterally acceptable solvent or pharmaceutically acceptable diluent is a cyclodextrin solution, and the cyclodextrin can be any cyclodextrin described herein, including any of the cyclodextrin or cyclodextrin derivatives listed in Tables A to D.

When the pharmaceutical compositions described herein are used to treat various disease, it can achieve better drug tolerance, higher drug exposure, longer duration of therapy, flexible handling, and improved stability than other commercially available compositions containing busulfan.

Some embodiments relate to a pharmaceutical composition described herein for use in a method of conditioning a subject for hematopoietic stem-cell transplantation, comprising administering the composition described herein to a subject in need thereof.

Some embodiments relate to a pharmaceutical composition described herein for use in a method of conditioning a subject for bone marrow transplantation, comprising administering the composition described herein to a subject in need thereof. This method can further comprise an additional step or steps of administering an immunosuppressive agent, and/or administering an additional dose or doses of T cell depleted bone marrow cells, to the subject. The foregoing methods are also useful for treating hemoglobinopathies, and/or inhibiting rejection of an organ or tissue transplant in the subject, as described herein.

Some embodiments relate to a pharmaceutical composition described herein for use in a method of treating leukemia, lymphoma, and myeloproliferative disorder, comprising administering the composition described herein to a subject in need thereof. Cancer cells may be eradicated from the body by administering the busulfan based compositions described herein. The busulfan compositions and process of preparing the same described herein represents a new and more effective tool for administering higher doses of such therapy while diminishing the risk of a toxic solvent associated with the administered treatment.

In some embodiments, the composition is administered intravenously or intramuscularly. In some embodiments, the composition or dilute thereof is administered as intravenous infusion. In some embodiments, the composition or dilute thereof is administered as intravenous push or bolus.

In some embodiments, the amount of adult dose of the busulfan is about 0.2, 0.4, 0.6, 0.8, 1.0, 1.2, or 1.4 mg, per kg of body weight. In some embodiments, the amount of adult dose is greater than about 0.2, 0.4, 0.6, 0.8, 1.0, 1.2, or 1.4 mg, per kg of body weight. In some embodiments, the amount of adult dose is less than about 0.8, 1, 1.2, 1.4 , 1.6, 1.8, or 2 mg, per kg of body weight. In some embodiments, the amount of adult dose of busulfan is in the range of about 0.2 to 2.0, about 0.4 to about 1.5, or about 0.6 to about 1 mg, per kg of body weight. In some embodiments, the busulfan composition is administered intravenously via a central venous catheter as a two-hour infusion every six hours for four consecutive days for a total of 16 doses.

### Kits for Intravenous Administration

Some embodiments include a kit comprising cyclodextrin (e.g., sulfoalkyl ether cyclodextrin) and busulfan. Some embodiments include a kit comprising cyclodextrin (e.g., sulfoalkyl ether cyclodextrin) and busulfan, wherein at least a major portion of the busulfan is complexed with the cyclodextrin. Some embodiments include a kit comprising 1) cyclodextrin (e.g., sulfoalkyl ether cyclodextrin) and busulfan, wherein at least a major portion of the busulfan is complexed with the cyclodextrin; and 2) a pharmaceutically acceptable solvent or diluent for reconstituting the busulfan composition. In some embodiments, the kits are used for parenteral administration. In some embodiments, the kits are used for intravenous administration.

In one embodiment, the busulfan and cyclodextrin wherein a major portion of the busulfan is complexed with cyclodextrin is provided in a first sterile container. In some embodiments, the pharmaceutically acceptable solvent that is used to reconstitute the busulfan solution is provided in a second sterile container. In some embodiments, the pharmaceutically acceptable solvent is an aqueous cyclodextrin solution provided in a separate container. In the case of solids for reconstitution, the composition containing busulfan and cyclodextrin are first prepared using the process described herein and then lyophilized into powder to be added to the container. In some embodiments, the solids are sterile crystalline products. In other embodiment, the solids are lyophiles. Non-limiting examples of agents to aid in lyophilization include sodium or potassium phosphates, citric acid, tartaric acid, gelatin, and carbohydrates such as dextrose, mannitol, and dextran. One embodiment includes non-sterile solids that are irradiated either before or after introduction into the container.

In the case of a liquid, the busulfan composition described herein may be dissolved or dispersed in a diluent or a pharmaceutically acceptable solvent ready for administration. In another embodiment, the solution or dispersion may be further diluted prior to administration. Some embodiments include providing the liquid in an IV bag. The liquid may be frozen to improve stability.

In one embodiment, the container includes other ingredients such as a pH adjuster, a solubilizing agent, or a dispersing agent. Non-limiting examples of pH adjusters include NaOH, sodium carbonate, sodium acetate, HCl, and citric acid.

In an alternative embodiment, the busulfan composition and the cyclodextrin used for reconstitution may be provided in a single sterile container. In some embodiments, the busulfan composition and the cyclodextrin used for reconstitution may be provided in separate containers. Each container may include a solid, solution, or dispersion. In such embodiments, the two containers may be provided in a single package or may be provided separately. In one embodiment, the composition described herein is provided as a solid while the parenterally acceptable solvent (e.g, water, saline, and cyclodextrin solution) is provided as a solution ready for reconstitution. In one embodiment, the composition described herein is provided as a solid while the parenterally acceptable agent (e.g, cyclodextrin) is provided first as a solid ready for reconstitution. In one such embodiment, the solution of the parenterally acceptable agent described herein is used as the parenterally acceptable agent diluent to reconstitute the other solid composition.

### Examples

### Example 1. Captisol^{®}-enabled Busulfan Phase Solubility data

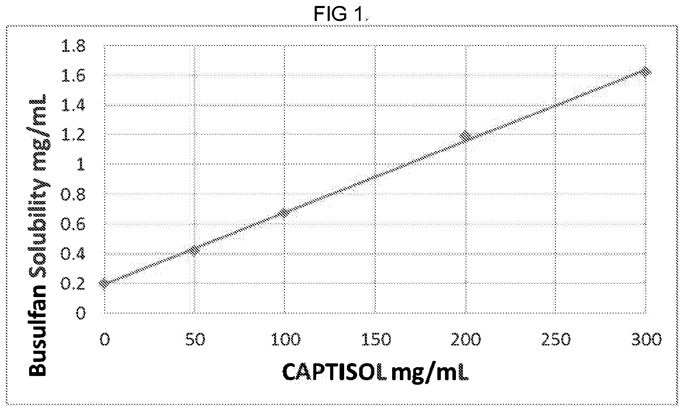

The phase solubility of busulfan was studied in sulfobutylether-β-cyclodextrin (Captisol^{®}) solutions, and the results are shown in Fig. 1. Busulfan was added to various Captisol^{®} solutions, and the mixture was measured with HPLC to determine the amount of busulfan dissolved in the solution after it reached equilibration after room temperature. It was also noted that the busulfan solution (1 mg/ml busulfan in 200 mg/ml Captisol^{®} solution and 2 mg/ml busulfan in 400 mg/ml Captisol^{®} solution) showed a stability of greater than 90% after keeping the solutions for 3 days at ambient temperature.

### Example 2. Captisol^{®}-enabled Busulfan formulation

A busulfan formulation was prepared by first dissolving busulfan in an organic solvent (e.g., acetone) and then combining it with Captisol^{®} aqueous solution. The mixture was then added into a rotary evaporator to remove the organic solvent. The solution remained clear after the acetone solvent was removed. The solution was then lyophilized to remove all water and produced a freeze-dried Captisol^{®} enabled busulfan powder. In one freeze-dried sample, the ratio of busulfan to Captisol by weight achieved 4:170, meaning the busulfan concentration was about 40 mg and the Captisol^{®} solution was about 1.7 g. Later, the lyophilized busulfan formulation was reconstituted and diluted with Captisol^{®} solution (100-150 mg/ml) to achieve infusion concentration. In one reconstituted sample, the busulfan powder quickly dissolved to form a clear solution having a busulfan concentration in the range of about 0.5 mg/ml to 2 mg/ml. It was noted that during the reconstitution step, 5mg/ml solubilized busulfan solution was achieved at a lower Captisol^{®} loading when compared with the method of directly combining busulfan and Captisol^{®} solution.

### Example 3. Stability of Captisol^{®}-enabled Busulfan formulation

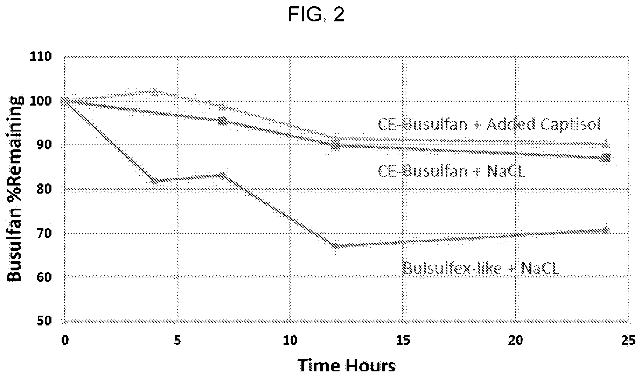

The stability of several Busulfan solutions was studied over time, and Figure 2 shows the precipitation amount of three busulfan compositions. The first busulfan composition was prepared using freeze drying/lyophilizing busulfan Captisol^{®} solution followed by reconstitution of the lyophilized mixture with Captisol^{®} solution; the second busulfan composition was prepared by combining busulfan and Captisol^{®} in a sodium chloride solution; and the third busulfan composition was a Bulsulfex^{®} (Otsuka Pharmaceutical; Tokyo, Japan: Each vial of BUSULFEX contains 60 mg (6 mg/mL) of busulfan, and the busulfan is dissolved in N,N-dimethylacetamide (DMA), 3.3 mL and Polyethylene Glycol 400, NF 6.7 mL) sample solution. The stability data showed that the first and the second Captisol^{®} enabled busulfan compositions had less precipitation than the Bulsulfex^{®} sample; and the precipitation of busulfan was delayed even further in the first busulfan formulation than the second busulfan composition. The first and second busulfan compositions both showed better stability than the Bulsulfex^{®} sample, with the first busulfan composition prepared by freeze-dry followed by reconstitution with additional Captisol^{®} showing the highest stability.

### Example 4. Test of Captisol^{®}-enabled Busulfan formulation

The stability of busulfan was studied in several formulations at three different temperatures. The first busulfan formulation was prepared using freeze drying/lyophilizing busulfan Captisol^{®} powder, which contained 60 mg of busulfan and 5.25 g of Captisol^{®}, followed by reconstitution of the lyophilized mixture with Captisol^{®} solution. The second busulfan formulation was prepared using freeze drying/lyophilizing busulfan Captisol^{®} powder, which contained 60 mg of busulfan and 5.25 g of Captisol^{®}, followed by reconstitution of the lyophilized mixture with saline diluent. The third busulfan composition was a Bulsilvex^{®} (Pierre Fabre Oncologie, Boulogne, France) reconstituted using 0.9% saline to form a final concentration of 0.55 mg/ml for Busulfan. The containers used for preparation were PP syringes (Becton Dickinson, Franklin Lakes, NJ). The sample solutions were aliquoted into smaller containers so that the solutions remained under the defined storage conditions throughout the evaluation period. For each of the storage temperature, a sample was processed and analyzed by HPLC-UV. Busulfan was detected by absorbance at 281 nm. In isocratic mode, a mobile phase consisting of acetonitrile (ACN), H2O, and trifluoroacetic acid (TFA) (proportions: 650/350/1, v/v/v) was run through the system at a flow rate of 2 mL/min. At time zero (T₀), the initial concentration (C₀) of the active substance was 100 %. The contents for each analysis time were thus determined on the basis of C₀.

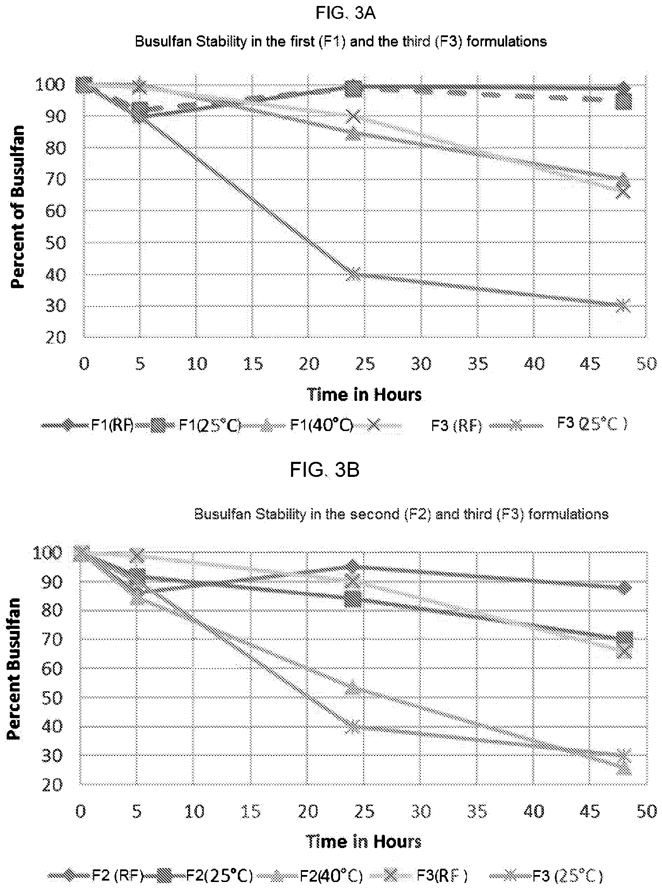

Figure 3A shows the stability of the first busulfan formulation at 2-8 °C, 25 °C, and 40 °C as well as the third busulfan formulation at 2-8 °C (RF) and 25 °C; and Figure 3B shows the stability of the second busulfan formulation at 2-8 °C(RF), 25 °C, and 40 °C as well as the third busulfan formulation at 2-8 °C (RF) and 25 °C. The stability data in both Figures 3A and 3B show that the Captisol^{®} enabled busulfan compositions, including the compositions reconstituted using Captisol^{®} solution and saline solution, were more stable than the Bulsilvex^{®} sample, which did not contain any Captisol^{®}.

### Example 5. Test of Captisol^{®}-enabled Busulfan formulation

Patients receive test doses of Captisol^{®} enabled Busulfan and IV Bulsulfex^{®} (Otsuka Pharmaceutical; Tokyo, Japan: Each vial of BUSULFEX contains 60 mg (6 mg/mL) of busulfan, and the busulfan is dissolved in N,N-dimethylacetamide (DMA), 3.3 mL and Polyethylene Glycol 400, NF 6.7 mL) at 0.8 mg/kg, with the two drug products alternating after a sufficient washout. The PK of Captisol^{®} enabled Busulfan is studied and compared with the IV Bulsulfex^{®} at the test dose. The safety and tolerability of high dose Captisol^{®} enabled Busulfan is also studied and compared with the IV Bulsulfex^{®} sample.

### Example 6. Dosing Regimen Study

Patients with chemo-sensitive relapsed or primary-refractory lymphoma undergoing a first autologous hematopoietic stem cell transplantation receive a test dose of Captisol^{®} enabled Busulfan (0.8 mg/kg) through IV given as a 2 hour continuous infusion on a day between Days -14 and -11. Busulfan exposure is determined as area under the concentration-time curve (AUC) using six whole blood samples obtained at defined intervals after the end of the infusion. Based on test PK, the remaining Busulfan dose is calculated to achieve a total AUC of 20,000 µM·min. One-fourth of this dose is given as a 3 hour infusion on Day -8, during which a second PKanalysis is done. The same daily Bu dose is administered on Days -7, -6, and -5 unless Day -8 PK results show total AUC outside the target ±20%. The busulfan infusion on Days -6 and -5 are modified if the second PK results predict that the total AUC would be less than 16000 µM·min or greater than 24000 µM·min. Etoposide 1.4g/m² is administered on Day -4 for 4 hour infusion, followed by cyclophosphamide 2.5 g/m²/day on Days -3 and -2.

Six serial blood samples are drawn after administration of the test dose (test PK) and on Day -8 (confirmatory PK). The busulfan concentrations are measured to determine the busulfan exposures as AUC using WinNonlin software, and to recommend individualized PK directed dosing. The target daily AUC during the conditioning regimen is calculated as (20000 µM·min - Test PK AUC)/4. The conditioning regimen daily dose is calculated as (Test PK dose/Test PK AUC)*Target daily AUC.

## Claims

1. A pharmaceutical composition, comprising a solid that comprises:
busulfan; and
a cyclodextrin; wherein at least about 25% of the busulfan in the composition is complexed with the cyclodextrin; optionally wherein at least 50% of the busulfan in the composition is complexed with the cyclodextrin; or wherein at least 90% of the busulfan in the composition is complexed with the cyclodextrin,
wherein the molar ratio of cyclodextrin to busulfan is less than 12,
wherein the cyclodextrin is a compound, or a mixture thereof, of Formula 1: 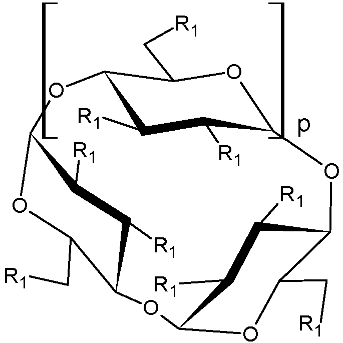
or a pharmaceutically acceptable salt thereof, wherein:
p is 4, 5, or 6, optionally wherein p is 5;
each R₁ is independently -OH or -O-( C₁-C₈ alkylene)-SO₃⁻-T, wherein at least one R₁ is -O-(C₁-C₈ alkylene)-SO₃⁻-T; and
T is hydrogen or a pharmaceutically acceptable cation;
provided that at least one R₁ is -OH.

2. The pharmaceutical composition of claim 1, wherein the molar ratio of cyclodextrin to busulfan is in the range of about 3 to about 10; optionally wherein the molar ratio of cyclodextrin to busulfan is less than about 9; optionally wherein the molar ratio of cyclodextrin to busulfan is less than about 5.

3. The pharmaceutical composition of claim 1 or claim 2, wherein the composition has a moisture content of less than 20%.

4. A reconstituted solution obtained by adding a pharmaceutically acceptable solvent to the composition of any one of claims 1 to 3, wherein the busulfan concentration is in the range of about 0.3 mg/ml to 3 mg/ml.

5. The reconstituted solution of claim 4, wherein the pharmaceutically acceptable solvent is saline; or wherein the pharmaceutically acceptable solvent is a cyclodextrin solution.

6. A pharmaceutical composition, comprising a clear aqueous solution according to claim 4 that comprises:
busulfan, wherein the concentration of busulfan is in the range of about 0.3 mg/ml to 3 mg/ml; and
a cyclodextrin, wherein the molar ratio of cyclodextrin to busulfan is less than about 12; optionally wherein the molar ratio of cyclodextrin to busulfan is less than about 10.

7. The pharmaceutical composition of claim 6, wherein the concentration of busulfan is in the range of about 0.5 mg/ml to about 2 mg/ml; optionally wherein the concentration of busulfan is about 0.55 mg/ml.

8. The pharmaceutical composition of claim 6 or claim 7, wherein at least about 50% of the busulfan in the composition is complexed with the cyclodextrin; and wherein the cyclodextrin is sulfoalkyl ether cyclodextrin.

9. The pharmaceutical composition of any one of claims 1-8, comprising less than 30% (w/w) of dimethylacetamide.

10. A sterilized container comprising the composition of any one of claims 1-9.

11. A process for preparing a busulfan composition, comprising:
combining busulfan and an organic solvent to provide a clear solution, optionally wherein the organic solvent is selected from dimethylacetamide, acetone, and any combination thereof, optionally wherein the organic solvent is acetone or wherein the organic solvent is dimethylacetamide;
combining the clear solution with a cyclodextrin to provide a first mixture, wherein combining the clear solution with a cyclodextrin comprises combining the clear solution with an aqueous cyclodextrin solution;
removing the organic solvent from the first mixture to provide a second mixture, optionally wherein the organic solvent is removed by evaporation; and
drying the second mixture to provide the busulfan composition, optionally wherein drying the second mixture comprises lyophilizing,
wherein the molar ratio of cyclodextrin to busulfan in the busulfan composition is less than 12,
wherein the cyclodextrin is a compound, or a mixture thereof, of Formula 1: 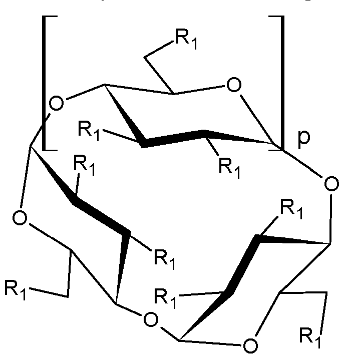
or a pharmaceutically acceptable salt thereof, wherein:
p is 4, 5, or 6, optionally wherein p is 5;
each R₁ is independently -OH or -O-( C₁-C₈ alkylene)-SO₃⁻-T, wherein at least one R₁ is -O-(C₁-C₈ alkylene)-SO₃⁻-T, optionally at least one R₁ is - OCH₂CH₂CH₂CH₂SO₃T or -OCH₂CH₂CH₂SO₃T; and
T is hydrogen or a pharmaceutically acceptable cation, optionally wherein T is independently hydrogen or sodium;
provided that at least one R₁ is -OH.

12. The process of claim 11, further comprising combining the busulfan composition with a parenterally acceptable solvent; optionally wherein the parenterally acceptable solvent is selected from water, saline, cyclodextrin solution, and any combination thereof.

13. The pharmaceutical composition of any one of claims 1 to 3 for use in therapy, wherein the therapy comprises;
reconstituting the pharmaceutical composition of any one of claims 1 to 3; and
administering the reconstituted pharmaceutical composition to a subject in need thereof; and
wherein the reconstituting comprises adding a parenterally acceptable solvent to the pharmaceutical composition;
wherein the parenterally acceptable solvent is selected from water, saline, cyclodextrin solution, and any combination thereof; optionally wherein the parenterally acceptable solvent is a cyclodextrin solution having a concentration in the range of about 50 mg/ml to about 200 mg/ml.

14. The pharmaceutical composition of any one of claims 1 to 3 for use in a method of conditioning a subject for hematopoietic stem-cell transplantation or for bone marrow transplantation, or for use in a method of treating leukemia, lymphoma, and myeloproliferative disorder.

## Patentansprüche

1. Pharmazeutische Zusammensetzung, umfassend einen Feststoff, der Folgendes umfasst:
Busulfan; und
ein Cyclodextrin; wobei mindestens etwa 25 % des Busulfans in der Zusammensetzung mit dem Cyclodextrin komplexiert sind; optional wobei mindestens 50 % des Busulfans in der Zusammensetzung mit dem Cyclodextrin komplexiert sind; oder wobei mindestens 90 % des Busulfans in der Zusammensetzung mit dem Cyclodextrin komplexiert sind,
wobei das Molverhältnis von Cyclodextrin zu Busulfan kleiner als 12 ist,
wobei das Cyclodextrin eine Verbindung, oder eine Mischung davon, der Formel 1: 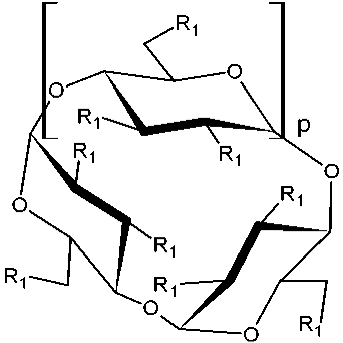
oder ein pharmazeutisch unbedenkliches Salzes davon ist, wobei:
p für 4, 5 oder 6 steht, optional wobei p für 5 steht;
jedes R₁ unabhängig -OH oder -O-(C₁-C₈-Alkylen)-SO₃ -T ist, wobei mindestens ein R₁ -O-(C₁-C₈-Alkylen)-SO₃ -T ist; und
T für Wasserstoff oder ein pharmazeutisch unbedenkliches Kation steht;
mit der Maßgabe, dass mindestens ein R₁ -OH ist.

2. Pharmazeutische Zusammensetzung nach Anspruch 1, wobei das Molverhältnis von Cyclodextrin zu Busulfan im Bereich von etwa 3 bis etwa 10 liegt; optional wobei das Molverhältnis von Cyclodextrin zu Busulfan kleiner als etwa 9 ist; optional wobei das Molverhältnis von Cyclodextrin zu Busulfan kleiner als etwa 5 ist.

3. Pharmazeutische Zusammensetzung nach Anspruch 1 oder Anspruch 2, wobei die Zusammensetzung einen Feuchtigkeitsgehalt von weniger als 20 % aufweist.

4. Rekonstituierte Lösung, erhalten durch Zugabe eines pharmazeutisch unbedenklichen Lösungsmittels zu der Zusammensetzung nach einem der Ansprüche 1 bis 3, wobei die Busulfan-Konzentration im Bereich von etwa 0,3 mg/ml bis 3 mg/ml liegt.

5. Rekonstituierte Lösung nach Anspruch 4, wobei das pharmazeutisch unbedenkliche Lösungsmittel Kochsalzlösung ist; oder wobei das pharmazeutisch unbedenkliche Lösungsmittel eine Cyclodextrinlösung ist.

6. Pharmazeutische Zusammensetzung, umfassend eine klare wässrige Lösung nach Anspruch 4, die Folgendes umfasst:
Busulfan, wobei die Konzentration von Busulfan im Bereich von etwa 0,3 mg/ml bis 3 mg/ml liegt; und
ein Cyclodextrin, wobei das Molverhältnis von Cyclodextrin zu Busulfan kleiner als etwa 12 ist; optional wobei das Molverhältnis von Cyclodextrin zu Busulfan kleiner als etwa 10 ist.

7. Pharmazeutische Zusammensetzung nach Anspruch 6, wobei die Konzentration von Busulfan im Bereich von etwa 0,5 mg/ml bis etwa 2 mg/ml liegt; optional wobei die Konzentration von Busulfan etwa 0,55 mg/ml beträgt.

8. Pharmazeutische Zusammensetzung nach Anspruch 6 oder Anspruch 7, wobei mindestens etwa 50 % des Busulfans in der Zusammensetzung mit dem Cyclodextrin komplexiert sind; und wobei das Cyclodextrin Sulfoalkylethercyclodextrin ist.

9. Pharmazeutische Zusammensetzung nach einem der Ansprüche 1-8, umfassend weniger als 30 % (W/W) Dimethylacetamid.

10. Sterilisierter Behälter, umfassend die Zusammensetzung nach einem der Ansprüche 1-9.

11. Verfahren zur Herstellung einer Busulfan-Zusammensetzung, umfassend:
Kombinieren von Busulfan und einem organischen Lösungsmittel, um eine klare Lösung bereitzustellen, wobei optional das organische Lösungsmittel ausgewählt ist aus Dimethylacetamid, Aceton und einer beliebigen Kombination davon, wobei optional das organische Lösungsmittel Aceton ist oder wobei das organische Lösungsmittel Dimethylacetamid ist;
Kombinieren der klaren Lösung mit einem Cyclodextrin, um eine erste Mischung bereitzustellen, wobei das Kombinieren der klaren Lösung mit einem Cyclodextrin das Kombinieren der klaren Lösung mit einer wässrigen Cyclodextrinlösung umfasst;
Entfernen des organischen Lösungsmittels aus der ersten Mischung, um eine zweite Mischung bereitzustellen, wobei optional das organische Lösungsmittel durch Verdampfen entfernt wird; und
Trocknen der zweiten Mischung, um die Busulfan-Zusammensetzung bereitzustellen, wobei optional das Trocknen der zweiten Mischung Lyophilisieren umfasst, wobei das Molverhältnis von Cyclodextrin zu Busulfan in der Busulfan-Zusammensetzung weniger als 12 beträgt, wobei das Cyclodextrin eine Verbindung, oder eine Mischung davon, der Formel 1: 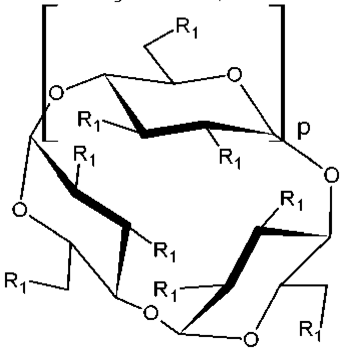
oder ein pharmazeutisch unbedenkliches Salzes davon ist, wobei:
p für 4, 5 oder 6 steht, optional wobei p für 5 steht; jedes R₁ unabhängig -OH oder -O-(C₁-C₈-Alkylen)-SO₃ -T ist, wobei mindestens ein R₁ -O-(C₁-C₈-Alkylen)-SO₃ -T ist, optional mindestens ein R₁ -OCH₂CH₂CH₂CH₂SO₃T oder - OCH₂CH₂CH₂SO₃T ist; und
T für Wasserstoff oder ein pharmazeutisch unbedenkliches Kation steht, optional wobei T auf unabhängige Weise für Wasserstoff oder Natrium steht;
mit der Maßgabe, dass mindestens ein R₁ -OH ist.

12. Verfahren nach Anspruch 11, ferner umfassend das Kombinieren der Busulfan-Zusammensetzung mit einem parenteral unbedenklichen Lösungsmittel; optional wobei das parenteral unbedenkliche Lösungsmittel aus Wasser, Kochsalzlösung, Cyclodextrin-Lösung und einer beliebigen Kombination davon ausgewählt ist.

13. Pharmazeutische Zusammensetzung nach einem der Ansprüche 1 bis 3 zur Verwendung in der Therapie, wobei die Therapie Folgendes umfasst:
Rekonstituieren der pharmazeutischen Zusammensetzung nach einem der Ansprüche 1 bis 3; und
Verabreichen der rekonstituierten pharmazeutischen Zusammensetzung an ein Individuum, das dies benötigt; und wobei das Rekonstituieren das Hinzufügen eines parenteral unbedenklichen Lösungsmittels zu der pharmazeutischen Zusammensetzung umfasst;
wobei das parenteral unbedenkliche Lösungsmittel ausgewählt ist aus Wasser, Kochsalzlösung, Cyclodextrinlösung und einer beliebigen Kombination davon; wobei das parenteral unbedenkliche Lösungsmittel optional eine Cyclodextrinlösung mit einer Konzentration im Bereich von etwa 50 mg/ml bis etwa 200 mg/ml ist.

14. Pharmazeutische Zusammensetzung nach einem der Ansprüche 1 bis 3 zur Verwendung in einem Verfahren zur Konditionierung eines Individuums für hämatopoetische Stammzelltransplantation oder für Knochenmarktransplantation oder zur Verwendung in einem Verfahren zur Behandlung von Leukämie, Lymphom und myeloproliferativer Störung.

## Revendications

1. Composition pharmaceutique, comprenant un solide qui comprend :
du busulfan ; et
une cyclodextrine ; dans laquelle au moins environ 25 % du busulfan dans la composition est complexé avec la cyclodextrine ; facultativement, dans laquelle au moins 50 % du busulfan dans la composition est complexé avec la cyclodextrine ; ou dans laquelle au moins 90 % du busulfan dans la composition est complexé avec la cyclodextrine,
dans laquelle le rapport molaire de la cyclodextrine sur le busulfan est inférieur à 12,
dans laquelle la cyclodextrine est un composé, ou un mélange de ceux-ci, de formule 1 : 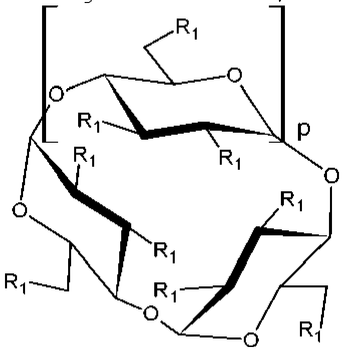
ou un sel pharmaceutiquement acceptable de celui-ci, dans laquelle :
p est 4, 5 ou 6, facultativement où p est 5 ;
chaque R₁ est indépendamment -OH ou -O-(alkylène en C₁-C₈)-SO₃ -T, où au moins un R₁ est -O-(alkylène en C₁-C₈)-SO₃ -T ; et
T est hydrogène ou un cation pharmaceutiquement acceptable ;
à condition qu'au moins un R₁ soit -OH.

2. Composition pharmaceutique selon la revendication 1, dans laquelle le rapport molaire de la cyclodextrine sur le busulfan est dans la plage d'environ 3 à environ 10 ; facultativement dans laquelle le rapport molaire de la cyclodextrine sur le busulfan est inférieur à environ 9 ; facultativement dans laquelle le rapport molaire de la cyclodextrine sur le busulfan est inférieur à environ 5.

3. Composition pharmaceutique selon la revendication 1 ou la revendication 2, dans laquelle la composition a une teneur en humidité inférieure à 20 %.

4. Solution reconstituée obtenue en ajoutant un solvant pharmaceutiquement acceptable à la composition de l'une quelconque des revendications 1 à 3, dans laquelle la concentration en busulfan est dans la plage d'environ 0,3 mg/ml à 3 mg/ml.

5. Solution reconstituée selon la revendication 4, dans laquelle le solvant pharmaceutiquement acceptable est une solution saline ; ou dans laquelle le solvant pharmaceutiquement acceptable est une solution de cyclodextrine.

6. Composition pharmaceutique, comprenant une solution aqueuse limpide selon la revendication 4, qui comprend :
du busulfan, dans laquelle la concentration de busulfan est dans la plage d'environ 0,3 mg/ml à 3 mg/ml ; et
une cyclodextrine, dans laquelle le rapport molaire de la cyclodextrine sur le busulfan est inférieur à environ 12 ; facultativement, dans laquelle le rapport molaire de la cyclodextrine sur le busulfan est inférieur à environ 10.

7. Composition pharmaceutique selon la revendication 6, dans laquelle la concentration de busulfan est dans la plage d'environ 0,5 mg/ml à environ 2 mg/ml ; facultativement, dans laquelle la concentration de busulfan est d'environ 0,55 mg/ml.

8. Composition pharmaceutique selon la revendication 6 ou la revendication 7, dans laquelle au moins environ 50 % du busulfan dans la composition est complexé avec la cyclodextrine ; et dans laquelle la cyclodextrine est un éther de sulfoalkyle de cyclodextrine.

9. Composition pharmaceutique selon l'une quelconque des revendications 1 à 8, comprenant moins de 30 % (p/p) de diméthylacétamide.

10. Récipient stérilisé comprenant la composition selon l'une quelconque des revendications 1 à 9.

11. Procédé de préparation d'une composition de busulfan comprenant :
la combinaison de busulfan et d'un solvant organique pour fournir une solution limpide, éventuellement dans lequel le solvant organique est choisi parmi le diméthylacétamide, l'acétone, et n'importe quelle combinaison de ceux-ci, éventuellement dans lequel le solvant organique est l'acétone ou dans lequel le solvant organique est le diméthylacétamide ;
la combinaison de la solution limpide avec une cyclodextrine pour fournir un premier mélange, dans lequel la combinaison de la solution limpide avec une cyclodextrine comprend la combinaison de la solution limpide avec une solution aqueuse de cyclodextrine ; l'élimination du solvant organique du premier mélange pour fournir un deuxième mélange, facultativement dans lequel le solvant organique est éliminé par évaporation ; et
le séchage du second mélange pour fournir la composition de busulfan, facultativement, le séchage du second mélange comprenant une lyophilisation,
dans lequel le rapport molaire de cyclodextrine sur busulfan dans la composition de busulfan est inférieur à 12,
dans laquelle la cyclodextrine est un composé, ou un mélange de ceux-ci, de formule 1 : 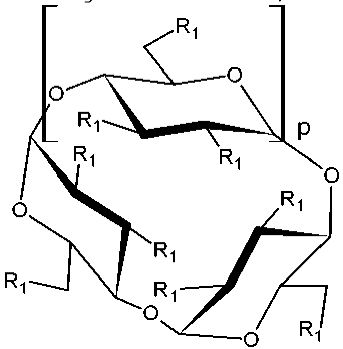
ou un sel pharmaceutiquement acceptable de celui-ci, dans laquelle :
p est 4, 5 ou 6, facultativement où p est 5 ;
chaque R₁ est indépendamment -OH ou -O-(alkylène en C₁-C₈)-SO₃ -T, où au moins un R₁ est -O-(alkylène en C₁-C₈)-SO₃ -T, facultativement au moins un R₁ est - OCH₂CH₂CH₂CH₂SO₃T ou -OCH₂CH₂CH₂SO₃T ; et
T est hydrogène ou un cation pharmaceutiquement acceptable, facultativement où T est indépendamment hydrogène ou sodium ;
à condition qu'au moins un R₁ soit -OH.

12. Procédé selon la revendication 11, comprenant en outre la combinaison de la composition de busulfan avec un solvant acceptable sur le plan parentéral ; facultativement, dans lequel le solvant acceptable sur le plan parentéral est choisi parmi l'eau, une solution saline, une solution de cyclodextrine et toute combinaison de celles-ci.

13. Composition pharmaceutique de l'une quelconque des revendications 1 à 3 pour utilisation en thérapie, la thérapie comprenant :
la reconstitution de la composition pharmaceutique selon l'une quelconque des revendications 1 à 3,
l'administration de la composition pharmaceutique reconstituée à un sujet en ayant besoin ; et
dans laquelle la reconstitution comprend l'ajout d'un solvant acceptable sur le plan parentéral à la composition pharmaceutique ;
dans laquelle le solvant acceptable sur le plan parentéral est choisi parmi l'eau, une solution saline, une solution de cyclodextrine, et une quelconque combinaison de celles-ci ; facultativement dans laquelle le solvant acceptable sur le plan parentéral est une solution de cyclodextrine ayant une concentration dans la plage d'environ 50 mg/ml à environ 200 mg/ml.

14. Composition pharmaceutique selon l'une quelconque des revendications 1 à 3, destinée à être utilisée dans un procédé de conditionnement d'un sujet pour une transplantation de cellules souches hématopoïétiques ou pour une transplantation de moelle osseuse, ou destinée à être utilisée dans un procédé de traitement de la leucémie, du lymphome et d'un trouble myéloprolifératif.
